# EUROPÄISCHE PATENTSCHRIFT

(11) **EP 3 394 136 B1**
(45) Veröffentlichungstag und Bekanntmachung des Hinweises auf die Patenterteilung: **25.08.2021**
(21) Anmeldenummer: 16822669.4
(22) Anmeldetag: 20.12.2016
(51) Int. Cl.: C09K 3/10, C08G 18/48, C08G 18/75, C08G 18/10, C08G 18/76, C09D 175/04, C08G 18/32, C08G 18/12

(54) **POLYALDIMIN UND HÄRTBARE POLYURETHANZUSAMMENSETZUNG**
POLYALDIMINE AND CURABLE POLYURETHANE COMPOSITION
POLYALDIMINE ET COMPOSITION DE POLYURETHANE DURCISSABLE

(30) Priorität: 21.12.2015 EP 15201649
(43) Veröffentlichungstag der Anmeldung: 31.10.2018
(73) Patentinhaber: Sika Technology AG, 6340 Baar (CH)
(72) Erfinder: BURCKHARDT, Urs, 8049 Zürich (CH); KRAMER, Andreas, 8006 Zürich (CH)
(74) Vertreter: Sika Patent Attorneys
(86) Internationale Anmeldenummer: PCT/EP2016/081979
(87) Internationale Veröffentlichungsnummer: WO 2017/108827

(56) Entgegenhaltungen:
- EP-A1- 2 017 260
- EP-A1- 2 857 378
- US-A- 5 087 661
- US-A1- 2003 096 893

## Beschreibung

### Technisches Gebiet

Die Erfindung betrifft Polyaldimine und Polyurethane, sowie Klebstoffe, Dichtstoffe und Beschichtungen.

### Stand der Technik

Härtbare Polyurethanzusammensetzungen, welche durch Reaktion von Isocyanatgruppen mit Hydroxylgruppen und/oder Feuchtigkeit bzw. Wasser vernetzen, werden in vielen technischen Anwendungen eingesetzt, beispielsweise als Klebstoffe, Dichtstoffe oder Beschichtungen in der Bau- und Fertigungsindustrie. Wenn solche Zusammensetzungen bei hoher Luftfeuchtigkeit und/oder erhöhter Temperatur eingesetzt werden, entstehen bei der Aushärtung oft störende Blasen durch freigesetztes Kohlendioxid-Gas, das nicht schnell genug gelöst oder abgeleitet wird. Um die Blasenbildung zu vermeiden, können den Zusammensetzungen chemisch blockierte Amine, sogenannte latente Härter, zugegeben werden, welche bei Kontakt mit Feuchtigkeit Aminogruppen freisetzen und dabei rasch und ohne Kohlendioxid-Bildung mit den Isocyanatgruppen vernetzen. Als latente Härter werden meist Verbindungen mit Aldimin-, Ketimin- oder Oxazolidin-Gruppen eingesetzt. Die bekannten latenten Härter bringen aber Nachteile mit sich. So können sie vorzeitige Vernetzungsreaktionen auslösen und damit die Lagerstabilität der Zusammensetzungen herabsetzen und/oder deren Aushärtung so stark beschleunigen, dass sich eine zu kurze Offenzeit und damit ein zu kurzes Verarbeitungsfenster ergibt. Zudem führen viele der bekannten latenten Härter bei der Aushärtung zu störenden Immissionen, verursacht durch leichtflüchtige, geruchsintensive Aldehyde oder Ketone, welche im latenten Härter als Blockierungsmittel dienen und durch Hydrolyse freigesetzt werden.

WO 00/64860 beschreibt Polyurethanzusammensetzungen enthaltend aromatische Aldimine von aromatischen Aldehyden. Diese Zusammensetzungen sind lagerstabil und weisen eine lange Topf- bzw. Offenzeit auf. Ihre Aushärtung verläuft aber meist unter starker Geruchsbildung und ist langsam und unvollständig, was zu Blasenbildung, klebrigen Oberflächen und/oder eingeschränkter Festigkeit oder Dehnbarkeit führen kann. Ausserdem müssen die Aldimine zur Herstellung der Zusammensetzungen erwärmt oder gelöst werden, da sie bei Raumtemperatur hochviskos oder fest sind.

US 5,087,661 beschreibt Polyaldimine mit einer Arylgruppe mit 6 bis 15 C-Atomen, sowie deren Verwendung als latente Härter in Polyurethanzusammensetzungen. Diese Polyaldimine sind einige Zeit nach der Herstellung bei Raumtemperatur mehrheitlich hochviskos oder fest und müssen deshalb zur Einarbeitung in eine Polyurethanzusammensetzung erwärmt oder gelöst werden. Die Zusammensetzungen sind oft wenig lagerstabil; insbesondere bei erhöhter Lagertemperatur und/oder bei Verwendung sterisch ungehinderter aromatischer Polyisocyanate wie MDI kann es schon nach kurzer Zeit zu einem starken Anstieg der Viskosität kommen, wodurch sie ihre Anwendbarkeit verlieren. Bei ihrer Verwendung treten zudem starke, lange anhaltende Geruchsbelastungen durch die freigesetzten geruchsintensiven Aldehyde auf.

WO 2004/013088 beschreibt Polyaldimine mit einer langkettigen, Estergruppen-haltigen tertiären Alkylgruppe, sowie deren Verwendung als latente Härter in Polyurethanzusammensetzungen. Diese Polyaldimine sind flüssig und niedrigviskos, aber temperatur- und feuchtigkeitsempfindlich und sehr reaktiv. Die Zusammensetzungen sind sehr lagerstabil und härten vollständig ohne Geruchsbildung aus, haben aber eine eher kurze Offenzeit. Wegen eingeschränkter Verträglichkeit der freigesetzten langkettigen Aldehyde mit dem ausgehärteten Polyurethan neigen sie zudem zu Weichmacher-Migration, was sich durch Ausschwitzen (Bleeding), oder Substratverfärbung oder Spannungsrissbildung im Substrat zeigen kann.

WO 2009/010522 beschreibt Polyaldimine mit einer aminhaltigen tertiären Alkylgruppe, sowie deren Verwendung als latente Härter in Polyurethanzusammensetzungen. Diese Polyaldimine sind ebenfalls flüssig und einfach verarbeitbar, aber nicht pH-neutral. Die Zusammensetzungen härten blasenfrei und geruchsarm aus, sind aber bei Verwendung aromatischer Polyisocyanate nicht lagerstabil und haben eine sehr kurze Offenzeit.

### Darstellung der Erfindung

Aufgabe der vorliegenden Erfindung ist es daher, latente Härter für Polyurethane zur Verfügung zu stellen, welche die Nachteile des Standes der Technik überwinden.

Überraschenderweise wurde gefunden, dass ein Polyaldimin der Formel (I) wie in Anspruch 1 beschrieben diese Aufgabe löst. Das Polyaldimin der Formel (I) ist geruchlos, pH-neutral, bei Raumtemperatur flüssig oder niedrigschmelzend und vergleichsweise niedrigviskos sowie wenig empfindlich gegenüber Wärme und Feuchtigkeit. Es kann somit einfach gelagert, transportiert, dosiert und verarbeitet werden. Mit Isocyanaten, auch besonders reaktiven, sterisch ungehinderten aromatischen Isocyanaten wie insbesondere MDI und Addukten davon ist es überraschend inert, auch bei erhöhter Temperatur, und ermöglicht damit besonders lagerstabile Zusammensetzungen. Unter Feuchtigkeitseinfluss reagiert das Polyaldimin der Formel (I) mit Isocyanaten relativ langsam, aber trotzdem vollständig und störungsfrei, wobei mittels geeigneter Katalysatoren die Reaktion aber auch schnell eingestellt werden kann. Dadurch wird eine grosse Bandbreite an Offenzeiten ermöglicht. Der bei der Hydrolyse freigesetzte Aldehyd ist unflüchtig, geruchlos und farblos und bewirkt somit weder Emissionen bzw. Geruchsimmissionen noch Verfärbungen. Überraschenderweise ist er in Polyurethanen so gut verträglich, dass er kaum Weichmacher-Migration verursacht. Trotz des langkettigen Substituenten am Aryl-Rest ist seine Verträglichkeit in Polyurethanen ähnlich gut wie jene von aromatischen Aldehyden ohne langkettige Substituenten, welche aber einen deutlichen bis starken, langanhaltenden Geruch verursachen. Die geringe Neigung zu Weichmachermigration ist überraschend. Polyaldimine, deren Abspalter ein hohes Molekulargewicht aufweisen, sind naturgemäss besonders kritisch in Bezug auf Weichmacher-migration nach der Aushärtung, da die eingesetzte Menge aufgrund des hohen Equivalentgewichts entsprechend hoch ist und somit eine hohe Menge an freigesetzem Aldehyd im ausgehärteten Material verbleibt. Zudem liesse der langkettige hydrophobe Alkyl- bzw. Alkoxy-Substituent, insbesondere bei verzweigter Struktur, im hydrophilen, Wasserstoffbrücken aufweisenden Polymergerüst von Polyurethanen eine eher schlechte Verträglichkeit erwarten.

Das Polyaldimin der Formel (I) besitzt aufgrund der speziellen Struktur mit den Resten A und Z eine besonders vorteilhafte Kombination von im Stand der Technik bisher nicht erreichten Eigenschaften. Es ist als latenter Härter für alle handelsüblichen Polyisocyanate verwendbar. Es ermöglicht geruchsfreie Polyurethanzusammensetzungen mit hervorragender Lagerstabilität und gut handhabbaren Verarbeitungszeiten, welche rasch und vollständig und ohne Blasenbildung und ganz ohne Geruchsimmissionen aushärten. Dabei entsteht ein blasenfreies Material mit nichtklebriger Oberfläche und guter Festigkeit, Dehnbarkeit und Beständigkeit, welches nicht zu Problemen mit Weichmacher-Migration wie Ausschwitzen (Bleeding), Substratverfärbung oder Spannungsrissbildung im Substrat neigt.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Polyaldimin der Formel (I), 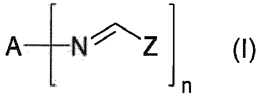 wobei
n für 2 oder 3 steht,
Z für einen mit einer Alkyl- oder Alkoxy-Gruppe substituierten Aryl-Rest mit insgesamt 12 bis 26 C-Atomen steht, und
A für einen n-wertigen aliphatischen oder cycloaliphatischen, gegebenenfalls Ether-Sauerstoff aufweisenden Kohlenwasserstoff-Rest mit einem Molekulargewicht im Bereich von 42 bis 6'000 g/mol, welcher über mindestens ein tertiäres oder quartäres C-Atom gebunden ist und/oder ein bi- oder tricyclisches Ringsystem enthält, steht,
dadurch gekennzeichnet, dass Z für einen Rest der Formel (II) steht, 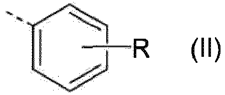 wobei R für einen verzweigten Alkyl-Rest mit 10 bis 14 C-Atomen steht.

Als "tertiäres C-Atom" wird ein C-Atom bezeichnet, welches nur an ein Wasserstoff-Atom gebunden ist.

Als "quartäres C-Atom" wird ein C-Atom bezeichnet, welches an kein Wasserstoff-Atom gebunden ist.

Eine gestrichelte Linie in den Formeln stellt jeweils die Bindung zwischen einem Substituenten und dem zugehörigen Molekülrest dar.

Als "primäre Aminogruppe" wird eine Aminogruppe bezeichnet, die an einen einzigen organischen Rest gebunden ist und zwei Wasserstoffatome trägt; als "sekundäre Aminogruppe" wird eine Aminogruppe bezeichnet, die an zwei organische Reste, welche auch gemeinsam Teil eines Rings sein können, gebunden ist und ein Wasserstoffatom trägt; und als "tertiäre Aminogruppe" wird eine Aminogruppe bezeichnet, die an drei organische Reste, welche auch zu zweit oder zu dritt Teil eines oder mehrerer Ringe sein können, gebunden ist und kein Wasserstoffatom trägt.

Mit "Poly" beginnende Substanznamen wie Polyamin, Polyol oder Polyisocyanat bezeichnen Substanzen, die formal zwei oder mehr der in ihrem Namen vorkommenden funktionellen Gruppen pro Molekül enthalten.

Als "primäres Polyamin" wird eine Verbindung mit mindestens zwei primären Aminogruppen bezeichnet.

Als "aromatisches Isocyanat" wird ein Isocyanat bezeichnet, dessen Isocyanatgruppen direkt an ein aromatisches C-Atom gebunden sind. Dementsprechend werden solche Isocyanatgruppen als "aromatische Isocyanatgruppen" bezeichnet.

Als "Molekulargewicht" wird die molare Masse (in Gramm pro Mol) eines Moleküls oder eines Molekül-Rests bezeichnet. Als "mittleres Molekulargewicht" wird das Zahlenmittel des Molekulargewichts (Mₙ) einer polydispersen Mischung von oligomeren oder polymeren Molekülen oder Molekül-Resten bezeichnet. Es wird üblicherweise mittels Gelpermeationschromatographie (GPC) gegen Polystyrol als Standard bestimmt.

Mit dem Begriff "Viskosität" wird die dynamische Viskosität oder Scherviskosität bezeichnet, welche durch das Verhältnis zwischen der Schubspannung und der Scherrate (Geschwindigkeitsgefälle) definiert ist und wie in DIN EN ISO 3219 beschrieben bestimmt wird.

Als "lagerstabil" oder "lagerfähig" wird eine Substanz oder eine Zusammensetzung bezeichnet, wenn sie bei Raumtemperatur in einem geeigneten Gebinde während längerer Zeit, typischerweise während mindestens 3 Monaten bis zu 6 Monaten und mehr, aufbewahrt werden kann, ohne dass sie sich in ihren Anwendungs- oder Gebrauchseigenschaften durch die Lagerung in einem für ihren Gebrauch relevanten Ausmass verändert.

Als "Raumtemperatur" wird eine Temperatur von 23 °C bezeichnet.

Z steht für einen Rest der Formel (II), 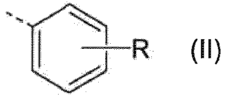 wobei R für einen verzweigten Alkyl-Rest mit 10 bis 14 C-Atomen steht.
R steht für einen verzweigten Alkyl-Rest mit 10 bis 14 C-Atomen. Ein solches Polyaldimin ist besonders reaktiv als latenter Härter in Polyurethanen.
R steht für einen verzweigten Alkyl-Rest. Ein solches Polyaldimin ist bei Raumtemperatur typischerweise flüssig und vergleichsweise niedrigviskos, was für seine Handhabung sehr vorteilhaft ist.
R steht für einen verzweigten Alkyl-Rest mit 10 bis 14 C-Atomen. Ein solches Polyaldimin ist besonders reaktiv, bei Raumtemperatur meist flüssig und vergleichsweise niedrigviskos.

Bevorzugt steht R in meta- oder para-Stellung, insbesondere in para-Stellung. Ein solches Polyaldimin ist besonders gut zugänglich. Ganz besonders bevorzugt steht R für einen Rest der Formel 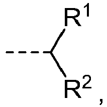 wobei R¹ und R² jeweils für einen Alkyl-Rest stehen und zusammen 9 bis 13 C-Atome aufweisen. Bevorzugt sind die Reste R¹ und R² jeweils linear.

Am meisten bevorzugt steht Z somit für einen Rest der Formel (IIa), 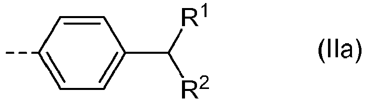 wobei R¹ und R² die genannten Bedeutungen aufweisen.

Die bevorzugten Reste Z sind besonders gut zugänglich und ermöglichen besonders geruchsarme Polyaldimine, welche bei Raumtemperatur insbesondere flüssig und besonders niedrigviskos sind.

A steht bevorzugt
- entweder für einen n-wertigen aliphatischen oder cycloaliphatischen Kohlenwasserstoff-Rest mit einem Molekulargewicht im Bereich von 42 bis 500 g/mol, welcher über mindestens ein tertiäres oder quartäres C-Atom gebunden ist und/oder ein bi- oder tricyclisches Ringsystem enthält
- oder für einen n-wertigen Polyoxyalkylen-Rest mit einem Molekulargewicht im Bereich von 170 bis 6'000 g/mol, bevorzugt 170 bis 2'000 g/mol, insbesondere 170 bis 470 g/mol, welcher über mindestens ein tertiäres C-Atom gebunden ist.

Ein solches Polyaldimin ist besonders einfach zugänglich.

Besonders bevorzugt ist A ausgewählt aus der Gruppe bestehend aus 1,2-Propylen, 1,3-Pentylen, 2-Methyl-1,5-pentylen, 2,2(4),4-Trimethyl-1,6-hexamethylen, 1,2-Cyclohexylen, (1,5,5-Trimethylcyclohexan-1-yl)methan-1,3, 4(2)-Methyl-1,3-cyclohexylen, Methylendicyclohexan-4-yl, Methylenbis(2-methylcyclohexan-4-yl), (Bicyclo[2.2.1]heptan-2,5(2,6)-diyl)dimethylen, (Tricyclo[5.2.1.0^{2,6}]-decan-3(4),8(9)-diyl)dimethylen, α,ω-Polyoxypropylen mit einem mittleren Molekulargewicht im Bereich von 170 bis 5'000 g/mol und Trimethylolpropan- oder Glycerin-gestartetem Tris(ω-polyoxypropylen) mit einem mittleren Molekulargewicht im Bereich von 330 bis 6'000 g/mol.

Die bevorzugten Reste A sind besonders gut zugänglich.

Davon bevorzugt ist 1,2-Cyclohexylen, (1,5,5-Trimethylcyclohexan-1-yl)methan-1,3, 4(2)-Methyl-1,3-cyclohexylen, Methylendicyclohexan-4-yl, (Bicyclo-[2.2.1]heptan-2,5(2,6)-diyl)dimethylen, (Tricyclo[5.2.1.0^{2,6}]decan-3(4),8(9)-diyl)-dimethylen, α,ω-Polyoxypropylen mit einem mittleren Molekulargewicht im Bereich von 170 bis 470 g/mol oder Trimethylolpropan- oder Glycerin-gestartetes Tris(ω-polyoxypropylen) mit einem mittleren Molekulargewicht im Bereich von 330 bis 450 g/mol.

Diese Polyaldimine ermöglichen Zusammensetzungen mit besonders guter Lagerstabilität und/oder besonders hohen Festigkeiten.

Davon besonders bevorzugt ist (1,5,5-Trimethylcyclohexan-1-yl)methan-1,3 oder (Bicyclo[2.2.1]heptan-2,5(2,6)-diyl)dimethylen oder (Tricyclo[5.2.1.0^{2,6}]decan-3(4),8(9)-diyl)dimethylen. Diese Polyaldimine der Formel (I) sind besonders niedrigviskos und ermöglichen besonders lagerstabile Zusammensetzungen mit besonders hoher Festigkeit.

Davon besonders bevorzugt ist weiterhin Methylendicyclohexan-4-yl. Diese Polyaldimine der Formel (I) ermöglichen besonders lagerstabile Zusammensetzungen mit besonders hohem Elastizitätsmodul.

Davon besonders bevorzugt ist weiterhin α,ω-Polyoxypropylen mit einem mittleren Molekulargewicht im Bereich von 170 bis 250 g/mol. Diese Polyaldimine der Formel (I) sind besonders niedrigviskos und ermöglichen besonders lagerstabile Zusammensetzungen mit besonders guter Dehnbarkeit.

Davon weiterhin besonders bevorzugt ist Trimethylolpropan-gestartetes Tris(ω-polyoxypropylen) mit einem mittleren Molekulargewicht im Bereich von 330 bis 450 g/mol. Diese Polyaldimine der Formel (I) sind besonders niedrigviskos und ermöglichen besonders lagerstabile Zusammensetzungen mit besonders hoher Vernetzungsdichte und Beständigkeit.

Die bevorzugten Polyaldimine der Formel (I) sind besonders gut zugänglich und/oder besonders niedrigviskos und/oder zusammen mit Isocyanaten besonders lagerstabil und/oder führen zu besonders guten mechanischen Eigenschaften oder Beständigkeiten und sind damit besonders geeignet als latente Härter für Klebstoffe, Dichtstoffe oder Beschichtungen auf Polyurethan-Basis.

Das Polyaldimin der Formel (I) wird bevorzugt erhalten aus der Umsetzung von mindestens einem Amin der Formel (III) mit mindestens einem Aldehyd der Formel (IV) in einer Kondensationsreaktion unter Freisetzung von Wasser.

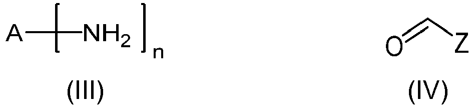

In den Formeln (III) und (IV) weisen n, A und Z die bereits genannten Bedeutungen auf.

Der Aldehyd der Formel (IV) wird dabei bevorzugt stöchiometrisch oder im stöchiometrischen Überschuss in Bezug auf die primären Aminogruppen eingesetzt. Auf diese Weise ist das Reaktionsprodukt weitgehend oder ganz frei von primären Aminogruppen.

Ein weiterer Gegenstand der Erfindung ist somit ein Reaktionsprodukt enthaltend mindestens ein Polyaldimin der Formel (I), erhalten aus der Umsetzung von mindestens einem Amin der Formel (III) mit mindestens einem Aldehyd der Formel (IV) in einer Kondensationsreaktion unter Freisetzung von Wasser, wobei der Aldehyd stöchiometrisch oder im stöchiometrischen Überschuss in Bezug auf die primären Aminogruppen vorhanden war.

Die Umsetzung wird vorteilhaft bei einer Temperatur im Bereich von 15 bis 120 °C, bevorzugt bei 20 bis 100°C, durchgeführt, gegebenenfalls in Anwesenheit eines Lösemittels. Das Kondensationswasser wird bevorzugt aus der Reaktionsmischung entfernt, entweder als Azeotrop mit einem geeigneten Lösemittel oder bevorzugt direkt durch Destillation, gegebenenfalls unter Vakuum. Gegebenenfalls wird bei der Umsetzung ein Katalysator eingesetzt, insbesondere ein Säure-Katalysator.

Besonders bevorzugt wird ohne Lösemittel gearbeitet und das Kondensationswasser mittels Anlegen von Vakuum aus der erwärmten Reaktionsmischung entfernt.

Ein solches Reaktionsprodukt kann ohne weitere Aufarbeitung als latenter Härter für Isocyanatgruppen aufweisende Zusammensetzungen verwendet werden.

Bevorzugt wird das Amin der Formel (III) mit dem Aldehyd der Formel (IV) zu einer Reaktionsmischung vereint, wobei der Aldehyd in Bezug auf die primären Aminogruppen stöchiometrisch oder im stöchiometrischen Überschuss vorhanden ist, und das Kondensationswasser mit einer geeigneten Methode aus der Reaktionsmischung entfernt wird, gegebenenfalls unter Erwärmen der Reaktionsmischung.

Als Amin der Formel (III) sind primäre aliphatische oder cycloaliphatische Di- oder Triamine geeignet, bei welchen mindestens eine primäre Aminogruppe an ein tertiäres oder quartäres C-Atom gebunden ist und/oder welche ein bi- oder tricyclisches Ringsystem enthalten.

Bevorzugt ist das Amin der Formel (III) ausgewählt aus der Gruppe bestehend aus 1,2-Propandiamin, 1,3-Pentandiamin, 1,5-Diamino-2-methylpentan (MPMD), 2,2(4),4-Trimethyl-1,6-hexandiamin (TMD), 1,2-Cyclohexandiamin, 1,3-Cyclohexandiamin, 1,4-Cyclohexandiamin, 1-Amino-3-aminomethyl-3,5,5-trimethylcyclohexan (Isophorondiamin oder IPDA), 4(2)-Methyl-1,3-cyclohexandiamin, Bis(4-aminocyclohexyl)methan, Bis(4-amino-3-methylcyclohexyl)methan, 2,5(2,6)-Bis(aminomethyl)bicyclo[2.2.1]heptan (Norbornandiamin oder NBDA), 3(4),8(9)-Bis(aminomethyl)tricyclo[5.2.1.0^{2,6}]decan (TCD Diamin), α,ω-Polyoxypropylendiamin mit einem mittleren Molekulargewicht im Bereich von 200 bis 4'000 g/mol, insbesondere die Jeffamine®-Typen D-230, D-400, XTJ-582, D-2000, XTJ-578 und D-4000 (alle von Huntsman), und Trimethylolpropan- oder Glycerin-gestartetem Tris(ω-polyoxypropylenamin) mit einem mittleren Molekulargewicht im Bereich von 380 bis 6'000 g/mol, insbesondere die Jeffamine®-Typen T-403, T-3000 und T-5000 (alle von Huntsman).

Diese Amine sind kommerziell besonders gut verfügbar.

Davon bevorzugt ist 1,2-Cyclohexandiamin, 1-Amino-3-aminomethyl-3,5,5-trimethylcyclohexan (Isophorondiamin oder IPDA), 4(2)-Methyl-1,3-cyclohexandiamin, Bis(4-aminocyclohexyl)methan, 2,5(2,6)-Bis(aminomethyl)bicyclo[2.2.1]-heptan (Norbornandiamin oder NBDA), 3(4),8(9)-Bis(aminomethyl)tricyclo-[5.2.1.0^{2,6}]decan (TCD Diamin), α,ω-Polyoxypropylen mit einem mittleren Molekulargewicht im Bereich von 200 bis 500 g/mol, insbesondere Jeffamine® D-230 oder Jeffamine® D-400, oder Trimethylolpropan- oder Glycerin-gestartetes Tris(ω-polyoxypropylen) mit einem mittleren Molekulargewicht im Bereich von 380 bis 500 g/mol, insbesondere Jeffamine® T-403.

Ein bevorzugter Aldehyd der Formel (IV) ist ein Aldehyd der Formel (IVa), wobei R die bereits beschriebenen Bedeutungen aufweist.

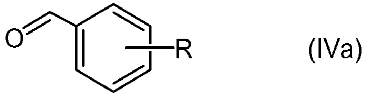

Ein besonders bevorzugter Aldehyd der Formel (IV) ist ein Aldehyd der Formel (IVb), wobei R¹ und R² die bereits beschriebenen Bedeutungen aufweisen.

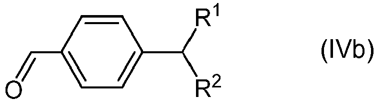

Als Aldehyd der Formel (IV) insbesondere bevorzugt sind 4-Decylbenzaldehyde, 4-Undecylbenzaldehyde, 4-Dodecylbenzaldehyde, 4-Tridecylbenzaldehyde oder 4-Tetradecylbenzaldehyde, bei welchen die Alkyl-Reste linear oder verzweigt, insbesondere verzweigt, sind.

Als Aldehyd der Formel (IV) am meisten bevorzugt ist ein Gemisch enthaltend 4-Decylbenzaldehyde, 4-Undecylbenzaldehyde, 4-Dodecylbenzaldehyde, 4-Tridecylbenzaldehyde und 4-Tetradecylbenzaldehyde, deren Alkyl-Reste mehrheitlich verzeigt sind.

Der Aldehyd der Formel (IV) ist insbesondere erhältlich aus der Formylierung von mindestens einem Alkyl- und/oder Alkoxy-substituierten aromatischen Kohlenwasserstoff mit Kohlenmonoxid unter Einwirkung eines Säurekatalysators. Als Säurekatalysator eignet sich beispielsweise das System HCl-AlCl₃ (Gattermann-Koch-Reaktion).

In einem bevorzugten Herstellprozess wird die Formylierung mit HF-BF₃ als Säurekatalysator durchgeführt. Dies ist vorteilhaft, da dieses Verfahren besonders selektiv verläuft und der Aldehyd der Formel (IV) aus dem Reaktionsgemisch ohne Hydrolyseschritt abgetrennt und der Katalysator wiederverwendet werden kann, womit eine aufwendige Produktaufarbeitung und Abfallentsorgung entfällt.

Besonders bevorzugt ist das Polyaldimin der Formel (I) ausgewählt aus der Gruppe bestehend aus N,N'-Bis(4-alkylbenzyliden)-1,2-cyclohexandiamin, N,N'-Bis(4-alkylbenzyliden)-3-aminomethyl-3,5,5-trimethylcyclohexylamin, N,N'-Bis(4-alkylbenzyliden)-4(2)-methyl-1,3-cyclohexandiamin, N,N'-Bis(4-alkylbenzyliden)-bis(4-aminocyclohexyl)methan, N,N'-Bis(4-alkylbenzyliden)-2,5(2,6)-bis(aminomethyl)bicyclo[2.2.1]heptan, N,N'-Bis(4-alkylbenzyliden)-3(4),8(9)-bis(aminomethyl)tricyclo[5.2.1.0^{2,6}]decan, N,N'-Bis(4-alkylbenzyliden)-polyoxypropylendiamin mit einem mittleren Molekulargewicht im Bereich von 650 bis 1050 g/mol und N,N',N"-Tris(4-alkylbenzyliden)polyoxypropylentriamin mit einem mittleren Molekulargewicht im Bereich von 1050 bis 1350 g/mol, wobei Alkyl jeweils für einen verzweigten Decyl-, Undecyl-, Dodecyl-, Tridecyl- oder Tetradecyl-Rest steht.

Das Polyaldimin der Formel (I) ist geruchlos, pH-neutral, bei Raumtemperatur flüssig oder niedrigschmelzend und vergleichsweise niedrigviskos sowie wenig empfindlich gegenüber Wärme und Feuchtigkeit. Es kann somit einfach gelagert, transportiert, dosiert und verarbeitet werden.

Bevorzugt stellt das Polyaldimin der Formel (I) ein Gemisch dar aus Polyaldiminen der Formel (I), bei welchen Z jeweils für einen Rest der Formel (II) steht und R dabei ausgewählt ist aus verzweigten Decyl-, Undecyl-, Dodecyl-, Tridecyl- und Tetradecyl-Resten.

Ein weiterer Gegenstand der Erfindung ist somit ein Gemisch aus Polyaldiminen der Formel (I), bei welchen Z jeweils für einen Rest der Formel (II) steht und R dabei ausgewählt ist aus verzweigten Decyl-, Undecyl-, Dodecyl-, Tridecyl- und Tetradecyl-Resten.

Ein solches Gemisch ist technisch besonders einfach zugänglich.

Ein weiterer Gegenstand der Erfindung ist die Verwendung von mindestens einem Polyaldimin der Formel (I) als latenter Härter für Isocyanatgruppen-haltige Zusammensetzungen.

Das Polyaldimin der Formel (I) weist vorteilhafte Eigenschaften für die beschriebene Verwendung auf. Es lässt sich ausgezeichnet in Isocyanatgruppen aufweisende Zusammensetzungen einmischen und zeigt kaum Neigung zu Separation. Es löst in Abwesenheit von Wasser bzw. Feuchtigkeit keine Vernetzungsreaktionen der Isocyanatgruppen aus und ermöglicht dadurch eine sehr gute Lagerstabilität. Bei Zutritt von Feuchtigkeit reagiert es rasch und vollständig über hydrolysierende Aldiminogruppen mit den Isocyanatgruppen, wobei dies weitgehend ohne konkurrierende Isocyanat-Hydrolyse und somit ohne Blasenbildung abläuft. Und schliesslich ist der bei der Aushärtung freigesetzte Aldehyd schwerflüchtig und verseifungsstabil, verursacht keine Geruchsbelastung, weist eine ausgezeichnete Verträglichkeit mit dem ausgehärteten Polymer auf und schwitzt kaum aus oder migriert in die Substrate.

Bevorzugt enthält die Isocyanatgruppen-haltige Zusammensetzung aromatische Isocyanatgruppen, welche insbesondere abgeleitet sind von 4,4'- oder 2,4'- oder 2,2'-Diphenylmethandiisocyanat oder beliebigen Gemischen dieser Isomeren (MDI) oder von 2,4- oder 2,6-Toluylendiisocyanat oder beliebigen Gemischen dieser Isomeren (TDI).

Zusammensetzungen mit aromatischen Isocyanatgruppen sind besonders kostengünstig, härten rasch aus und ermöglichen hohe Festigkeiten. Die Lagerstabilität, Offenzeit sowie die störungsfreie Aushärtung solcher Zusammensetzungen sind aber wesentlich kritischer, da aromatische Isocyanatgruppen deutlich reaktiver sind als aliphatische.

Zusammensetzungen auf Basis von MDI sind besonders geeignet als Klebstoff. Sie sind besonders kostengünstig und besonders reaktiv und ermöglichen besonders hohe Festigkeiten und Beständigkeiten. Allerdings sind sie auch besonders anspruchsvoll in Bezug auf Lagerstabilität, Offenzeit und Blasenbildung. Überraschenderweise werden mit dem Polyaldimin der Formel (I) sehr gute Lagerstabilitäten erhalten, bei einer langen Offenzeit und trotzdem zuverlässig unterdrückter Blasenbildung. Ähnliche Polyaldimine, welche nicht der Formel (I) entsprechen, ergeben typischerweise MDI-basierte einkomponentige Zusammensetzungen mit begrenzter Lagerstabilität und meist kurzer Offenzeit.

Zusammensetzungen auf Basis von TDI sind besonders kostengünstig und besonders niedrigviskos. Sie ermöglichen tiefe Elastizitätsmoduli und somit besonders weichelastische Eigenschaften. Sie sind besonders geeignet für Anwendungen, bei welchen Bewegungen mit geringer Kraftübertragung überbrückt werden sollen, wie beispielsweise Dichtstoffe für Dilatationsfugen oder Beschichtungen mit ausgeprägt rissüberbrückenden Eigenschaften. Allerdings sind sie auch besonders anspruchsvoll in Bezug auf Vollständigkeit der Aushärtung und Weichmacher-Migration, insbesondere bei niedrig eingestelltem Elastizitätsmodul, was sich in einer erhöhter Oberflächenklebrigkeit äussern kann. Überraschenderweise werden mit dem Polyaldimin der Formel (I) besonders tiefe Elastizitätsmoduli bei sehr geringer Oberflächenklebrigkeit erhalten, während mit ähnlichen Polyaldiminen, welche nicht der Formel (I) entsprechen, typischerweise deutlich höhere Elastizitätsmoduli erhalten werden.

Besonders bevorzugt ist die Verwendung von mindestens einem Polyaldimin der Formel (I) als latenter Härter für Isocyanatgruppen-haltige Zusammensetzungen, wobei die Isocyanatgruppen teilweise oder vollständig abgeleitet sind von 4,4'- oder 2,4'- oder 2,2'-Diphenylmethandiisocyanat oder beliebigen Gemischen dieser Isomeren (MDI). Diese Zusammensetzungen ermöglichen besonders hohe Festigkeiten und Beständigkeiten, sind aber aufgrund der sehr hohen Reaktivität von MDI auch besonders anspruchsvoll in Bezug auf die Lagerstabilität. Überraschenderweise zeigen die Polyaldimine der Formel (I) in Isocyanatgruppen-haltigen Zusammensetzungen auf der Basis von MDI aber eine sehr gute Lagerstabilität, während ähnliche Polyaldimine, welche auf nicht der Formel (III) entsprechenden Aminen, wie beispielsweise Hexamethylendiamin, 1,3-Bis(aminomethyl)cyclohexan (BAC) oder 1,3-Bis(aminomethyl)benzol (MXDA) basieren, zusammen mit MDI und Addukten davon nicht lagerstabil sind.

Ein weiterer Gegenstand der Erfindung ist eine Zusammensetzung umfassend
- mindestens ein Polyaldimin der Formel (I) und
- mindestens ein Polyisocyanat und/oder mindestens ein Isocyanatgruppen-haltiges Polyurethanpolymer.

Als Polyaldimin der Formel (I) geeignet sind die vorgängig beschriebenen.

Als Polyisocyanat geeignet ist insbesondere ein im Handel erhältliches Polyisocyanat, insbesondere
- aromatische Di- oder Triisocyanate, bevorzugt 4,4'- oder 2,4'- oder 2,2'-Diphenylmethandiisocyanat oder beliebigen Gemischen dieser Isomeren (MDI), 2,4- oder 2,6-Toluylendiisocyanat oder beliebige Gemische dieser Isomeren (TDI), Gemische aus MDI und MDI-Homologen (polymeres MDI oder PMDI), 1,3- oder 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diiso-cyanatodiphenyl (TODI), Dianisidindiisocyanat (DADI), Tris-(4-isocyanatophenyl)methan oder Tris-(4-isocyanatophenyl)thiophosphat; bevorzugt MDI oder TDI;
- aliphatische, cycloaliphatische oder arylaliphatische Di- oder Triisocyanate, bevorzugt 1,4-Tetramethylendiisocyanat, 2-Methylpentamethylen-1,5-diisocyanat, 1,6-Hexamethylendiisocyanat (HDI), 2,2,4- und/oder 2,4,4-Trimethyl-1,6-hexamethylendiisocyanat (TMDI), 1,10-Decamethylendiisocyanat, 1,12-Dodecamethylendiisocyanat, Lysin- oder Lysinesterdiisocyanat, Cyclohexan-1,3- oder -1,4-diisocyanat, 1-Methyl-2,4- und/oder -2,6-diisocyanatocyclohexan (H₆TDI), 1-Isocyanato-3,3,5-trimethyl-5-isocyanatomethylcyclohexan (IPDI), Perhydro-2,4'- und/oder -4,4'-diphenylmethandiisocyanat (H₁₂MDI), 1,3- oder 1,4-Bis-(isocyanatomethyl)cyclohexan, m- oder p-Xylylendiisocyanat, Tetramethyl-1,3- oder -1,4-xylylendiisocyanat, 1,3,5-Tris-(isocyanatomethyl)benzol, Bis-(1-Isocyanato-1-methylethyl)naphthalin, Dimer- oder Trimerfettsäureisocyanate wie insbesondere 3,6-Bis-(9-isocyanatononyl)-4,5-di(1-heptenyl)cyclohexen (Dimeryldiisocyanat); bevorzugt H₁₂MDI oder HDI oder IPDI;.
- Oligomere oder Derivate der genannten Di- oder Triisocyanate, insbesondere abgeleitet von HDI, IPDI, MDI oder TDI, insbesondere Oligomere enthaltend Uretdion- oder Isocyanurat- oder Iminooxadiazindion-Gruppen oder verschiedene dieser Gruppen; oder zwei- oder mehrwertige Derivate enthaltend Ester- oder Harnstoff- oder Urethan- oder Biuret- oder Allophanat- oder Carbodiimid- oder Uretonimin- oder Oxadiazintrion-Gruppen oder verschiedene dieser Gruppen. Solche Polyisocyanate stellen in der Praxis üblicherweise Gemische von Substanzen mit unterschiedlichen Oligomerisierungsgraden und/oder chemischen Strukturen dar. Insbesondere weisen sie eine mittlere NCO-Funktionalität von 2.1 bis 4.0 auf.

Ein besonders bevorzugtes Polyisocyanat ist HDI, IPDI, H₁₂MDI, TDI, MDI oder eine bei Raumtemperatur flüssige Form von MDI.

Eine bei Raumtemperatur flüssige Form von MDI stellt entweder durch partielle chemische Modifizierung - insbesondere Carbodiimidisierung bzw. Uretoniminbildung oder Adduktbildung mit Polyolen - verflüssigtes 4,4'-MDI dar, oder es ist ein durch Abmischen gezielt herbeigeführtes oder durch den Herstellungsprozess bedingtes Gemisch von 4,4'-MDI mit anderen MDI-Isomeren (2,4'-MDI und/oder 2,2'-MDI), und/oder MDI-Oligomeren und/oder MDI-Homologen (PMDI).

Am meisten bevorzugt ist MDI oder eine bei Raumtemperatur flüssige Form von MDI.

Ein geeignetes Isocyanatgruppen-haltiges Polyurethanpolymer wird insbesondere erhalten aus der Umsetzung von mindestens einem Polyol mit einer überstöchiometrischen Menge von mindestens einem Polyisocyanat. Die Umsetzung wird bevorzugt unter Feuchtigkeitsausschluss bei einer Temperatur im Bereich von 50 bis 160 °C durchgeführt, gegebenenfalls in Anwesenheit geeigneter Katalysatoren. Das NCO/OH-Verhältnis liegt bevorzugt im Bereich von 1.3/1 bis 2.5/1. Das nach der Umsetzung der OH-Gruppen im Reaktionsgemisch verbleibende Polyisocyanat, insbesondere monomeres Diisocyanat, kann entfernt werden, insbesondere mittels Destillation, was im Fall eines hohen NCO/OH-Verhältnisses bevorzugt ist. Das erhaltene Polyurethanpolymer weist bevorzugt einen Gehalt an freien Isocyanatgruppen im Bereich von 0.5 bis 10 Gewichts-%, insbesondere 1 bis 5 Gewichts-%, besonders bevorzugt 1 bis 3 Gewichts-%, auf. Gegebenenfalls kann das Polyurethanpolymer unter Mitverwendung von Weichmachern oder Lösemitteln hergestellt werden, wobei die verwendeten Weichmacher oder Lösemittel keine gegenüber Isocyanaten reaktive Gruppen enthalten.

Als Polyisocyanat zur Herstellung eines Isocyanatgruppen-haltigen Polyurethanpolymers bevorzugt sind die bereits genannten Polyisocyanate, insbesondere die Diisocyanate, insbesondere MDI, TDI, IPDI, HDI oder H₁₂MDI. Am meisten bevorzugt ist MDI. Damit werden besonders hohe Festigkeiten und Beständigkeiten erhalten.

Geeignete Polyole sind handelsübliche Polyole oder Mischungen davon, insbesondere
- Polyetherpolyole, insbesondere Polyoxyalkylendiole und/oder Polyoxyalkylentriole, insbesondere Polymerisationsprodukte von Ethylenoxid oder 1,2-Propylenoxid oder 1,2- oder 2,3-Butylenoxid oder Oxetan oder Tetrahydrofuran oder Mischungen davon, wobei diese mit Hilfe eines Startermoleküls mit zwei oder mehreren aktiven Wasserstoffatomen polymerisiert sein können, insbesondere einem Startermolekül wie Wasser, Ammoniak oder einer Verbindung mit mehreren OH- oder NH-Gruppen wie beispielsweise 1,2-Ethandiol, 1,2- oder 1,3-Propandiol, Neopentylglykol, Diethylenglykol, Triethylenglykol, die isomeren Dipropylenglykole oder Tripropylenglykole, die isomeren Butandiole, Pentandiole, Hexandiole, Heptandiole, Octandiole, Nonandiole, Decandiole, Undecandiole, 1,3- oder 1,4-Cyclohexandimethanol, Bisphenol A, hydriertes Bisphenol A, 1,1,1-Trimethylolethan, 1,1,1-Trimethylolpropan, Glycerin oder Anilin, oder Mischungen der vorgenannten Verbindungen. Ebenfalls geeignet sind Polyetherpolyole mit darin dispergierten Polymerpartikeln, insbesondere solche mit Styrol-Acrylnitril-Partikeln (SAN) oder Polyharnstoff- bzw. Polyhydrazodicarbonamid-Partikeln (PHD). Bevorzugte Polyetherpolyole sind Polyoxypropylendiole oder Polyoxypropylentriole, oder sogenannte Ethylenoxid-terminierte (EO-endcapped) Polyoxypropylendiole oder -triole. Letztere sind Polyoxyethylen-polyoxypropylen-Mischpolyole, die insbesondere dadurch erhalten werden, dass Polyoxypropylendiole oder -triole nach Abschluss der Polypropoxylierungsreaktion mit Ethylenoxid weiter alkoxyliert werden und dadurch schliesslich primäre Hydroxylgruppen aufweisen.

Bevorzugte Polyetherpolyole weisen einen Ungesättigtheitsgrad von weniger als 0.02 mEq/g, insbesondere weniger als 0.01 mEq/g auf.
- Polyesterpolyole, auch Oligoesterole genannt, hergestellt nach bekannten Verfahren, insbesondere der Polykondensation von Hydroxycarbonsäuren bzw. Lactonen oder der Polykondensation von aliphatischen und/oder aromatischen Polycarbonsäuren mit zwei- oder mehrwertigen Alkoholen. Bevorzugt sind Polyesterdiole aus der Umsetzung von zweiwertigen Alkoholen wie insbesondere 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1-Trimethylolpropan oder Mischungen der vorgenannten Alkohole, mit organischen Dicarbonsäuren oder deren Anhydride oder Ester, wie insbesondere Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure oder Hexahydrophthalsäure oder Mischungen der vorgenannten Säuren, oder Polyesterpolyole aus Lactonen wie insbesondere ε-Caprolacton. Besonders bevorzugt sind Polyesterpolyole aus Adipinsäure oder Sebacinsäure oder Dodecandicarbonsäure und Hexandiol oder Neopentylglykol.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen, insbesondere Polyetherpolyesterpolyole.
- Polyacrylat- und Polymethacrylatpolyole.
- Polyhydroxyfunktionelle Fette und Öle, beispielsweise natürliche Fette und Öle, inbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie beispielsweise polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen und Isopren, beispielsweise polyhydroxyfunktionelle Acrylonitril/ Butadien-Copolymere, wie sie beispielsweise aus Epoxiden oder Aminoalkoholen und carboxylterminierten Acrylonitril/Butadien-Copolymeren (beispielsweise kommerziell erhältlich unter dem Namen Hypro® CTBN oder CTBNX oder ETBN von Emerald Performance Materials) hergestellt werden können; sowie hydrierte polyhydroxyfunktionelle Polymere oder Copolymere von Dienen.

Geeignet sind insbesondere auch Mischungen von Polyolen.

Bevorzugt sind Polyetherpolyole, Polyesterpolyole, Polycarbonatpolyole, Poly-(meth)acrylatpolyole oder Polybutadienpolyole.

Besonders bevorzugt sind Polyetherpolyole, Polyesterpolyole, insbesondere aliphatische Polyesterpolyole, oder Polycarbonatpolyole, insbesondere aliphatische Polycarbonatpolyole.

Am meisten bevorzugt sind Polyetherpolyole, insbesondere Polyoxypropylendi- oder -triole oder Ethylenoxid-terminierte Polyoxypropylendi- oder -triole. Bevorzugt sind Polyole mit einem mittleren Molekulargewicht im Bereich von 400 bis 20'000 g/mol, bevorzugt von 1'000 bis 15'000 g/mol.

Bevorzugt sind Polyole mit einer mittleren OH-Funktionalität im Bereich von 1.6 bis 3.

Bevorzugt sind bei Raumtemperatur flüssige Polyole.

Bei der Herstellung eines Isocyanatgruppen-haltigen Polyurethanpolymers können auch Anteile von zwei- oder mehrfunktionellen Alkoholen mitverwendet werden, insbesondere 1,2-Ethandiol, 1,2-Propandiol, 1,3-Propandiol, 2-Methyl-1,3-propandiol, 1,2-Butandiol, 1,3-Butandiol, 1,4-Butandiol, 1,3-Pentandiol, 1,5-Pentandiol, 3-Methyl-1,5-pentandiol, Neopentylglykol, Dibromneopentylglykol, 1,2-Hexandiol, 1,6-Hexandiol, 1,7-Heptandiol, 1,2-Octandiol, 1,8-Octandiol, 2-Ethyl-1,3-hexandiol, Diethylenglykol, Triethylenglykol, Dipropylenglykol, Tripropylenglykol, 1,3- oder 1,4-Cyclohexandimethanol, ethoxyliertes Bisphenol-A, propoxyliertes Bisphenol-A, Cyclohexandiol, hydriertes Bisphenol-A, Dimerfettsäurealkohole, 1,1,1-Trimethylolethan, 1,1,1-Trimethylolpropan, Glycerin, Pentaerythritol, Zuckeralkohole wie insbesondere Xylit, Sorbit oder Mannit oder Zucker wie insbesondere Saccharose oder alkoxylierte Derivate der genannten Alkohole oder Mischungen der genannten Alkohole.

Das Isocyanatgruppen-haltige Polyurethanpolymer weist bevorzugt ein mittleres Molekulargewicht im Bereich von 1'500 bis 20'000 g/mol, insbesondere 2'000 bis 15'000 g/mol,auf.

Bevorzugt ist es bei Raumtemperatur flüssig.

Bevorzugt enthält die Zusammensetzung mindestens ein Isocyanatgruppen-haltiges Polyurethanpolymer.

Bevorzugt weist das Isocyanatgruppen-haltige Polyurethanpolymer aromatische Isocyanatgruppen auf, welche insbesondere abgeleitet sind von 4,4'- oder 2,4'- oder 2,2'-Diphenylmethandiisocyanat oder beliebigen Gemischen dieser Isomeren (MDI) oder von 2,4- oder 2,6-Toluylendiisocyanat oder beliebigen Gemischen dieser Isomeren (TDI).

Am meisten bevorzugt umfasst die Zusammensetzung ein Isocyanatgruppen-haltiges Polyurethanpolymer, dessen Isocyanatgruppen teilweise oder vollständig abgeleitet sind von 4,4'- oder 2,4'- oder 2,2'-Diphenylmethandiisocyanat oder beliebigen Gemischen dieser Isomeren (MDI). Damit werden besonders hohe Festigkeiten und Beständigkeiten erreicht.

Zusätzlich zu einem Isocyanatgruppen-haltigen Polyurethanpolymer kann die Zusammensetzung weiterhin mindestens ein Diisocyanat und/oder ein Oligomer oder Polymer eines Diisocyanates enthalten, insbesondere eine bei Raumtemperatur flüssige Form von MDI oder PMDI oder ein IPDI-Isocyanurat oder TDI-Oligomer oder ein gemischtes Isocyanurat auf Basis TDI/HDI oder ein HDI-Oligomer.

Bevorzugt enthält die Zusammensetzung neben mindestens einem Polyaldimin der Formel (I) und mindestens einem Polyisocyanat zusätzlich einen oder mehrere weitere Bestandteile, welche insbesondere ausgewählt sind aus Katalysatoren, Füllstoffen, Weichmachern und Lösemitteln.

Geeignete Katalysatoren sind insbesondere Katalysatoren für die Hydrolyse der Aldiminogruppen, insbesondere organische Säuren, insbesondere Carbonsäuren wie 2-Ethylhexansäure, Laurinsäure, Stearinsäure, Isostearinsäure, Ölsäure, Neodecansäure, Benzoesäure, Salicylsäure oder 2-Nitrobenzoesäure, organische Carbonsäureanhydride wie Phthalsäureanhydrid, Hexahydrophthalsäureanhydrid oder Hexahydromethylphthalsäureanhydrid, Silylester von Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester. Besonders bevorzugt sind Carbonsäuren, insbesondere aromatische Carbonsäuren wie Benzoesäure, 2-Nitrobenzoesäure oder insbesondere Salicylsäure.

Geeignete Katalysatoren sind weiterhin Katalysatoren für die Beschleunigung der Reaktion von Isocyanatgruppen, insbesondere Organozinn(IV)-Verbindungen wie insbesondere Dibutylzinndiacetat, Dibutylzinndilaurat, Dibutylzinndichlorid, Dibutylzinndiacetylacetonat, Dimethylzinndilaurat, Dioctylzinndiacetat, Dioctylzinndilaurat oder Dioctylzinndiacetylacetonat, Komplexverbindungen von Bismut(III) oder Zirkonium(IV), insbesondere mit Liganden ausgewählt aus Alkoholaten, Carboxylaten, 1,3-Diketonaten, Oxinat, 1,3-Ketoesteraten und 1,3-Ketoamidaten, oder tertiäre Aminogruppen enthaltende Verbindungen wie insbesondere 2,2'-Dimorpholinodiethylether (DMDEE).

Geeignet sind insbesondere auch Kombinationen von verschiedenen Katalysatoren.

Geeignete Füllstoffe sind insbesondere gemahlene oder gefällte Calciumcarbonate, welche gegebenenfalls mit Fettsäuren, insbesondere Stearaten, beschichtet sind, Baryte (Schwerspate), Quarzmehle, Quarzsande, Dolomite, Wollastonite, Kaoline, calcinierte Kaoline, Schichtsilikate wie Glimmer oder Talk, Zeolithe, Aluminiumhydroxide, Magnesiumhydroxide, Kieselsäuren inklusive hochdisperse Kieselsäuren aus Pyrolyseprozessen, Zemente, Gipse, Flugaschen, industriell hergestellte Russe, Graphit, Metall-Pulver, beispielsweise von Aluminium, Kupfer, Eisen, Silber oder Stahl, PVC-Pulver oder Hohlkugeln.

Geeignete Weichmacher sind insbesondere Carbonsäureester wie Phthalate, insbesondere Diisononylphthalat (DINP), Diisodecylphthalat (DIDP) oder Di(2-propylheptyl)phthalat (DPHP), hydrierte Phthalate, insbesondere hydriertes Diisononylphthalat (DINCH), Terephthalate, insbesondere Dioctylterephthalat, Trimellitate, Adipate, insbesondere Dioctyladipat, Azelate, Sebacate, Benzoate, Glykolether, Glykolester, organische Phosphor- oder Sulfonsäureester, Polybutene, Polyisobutene oder von natürlichen Fetten oder Ölen abgeleitete Weichmacher, insbesondere epoxidiertes Soja- oder Leinöl.

Geeignete Lösemittel sind insbesondere Aceton, Methylethylketon, Methyl-n-propylketon, Diisobutylketon, Methylisobutylketon, Methyl-n-amylketon, Methylisoamylketon, Acetylaceton, Mesityloxid, Cyclohexanon, Methylcyclohexanon, Ethylacetat, Propylacetat, Butylacetat, n-Butylpropionat, Diethylmalonat, 1-Methoxy-2-propylacetat, Ethyl-3-ethoxypropionat, Diisopropylether, Diethylether, Dibutylether, Diethylenglykoldiethylether, Ethylenglykoldiethylether, Ethylenglykolmonopropylether, Ethylenglykolmono-2-ethylhexylether, Toluol, Xylol, Heptan, Octan, Naphtha, White Spirit, Petrolether oder Benzin, insbesondere Solvesso™-Typen (von Exxon), weiterhin Methylenchlorid, Propylencarbonat, Butyrolacton, N-Methylpyrrolidon oder N-Ethylpyrrolidon.

Die Zusammensetzung kann weitere für Polyurethanzusammensetzungen gebräuchliche Zusätze enthalten. Insbesondere können die folgenden Hilfs- und Zusatzstoffe vorhanden sein:
- anorganische oder organische Pigmente, insbesondere Titandioxid, Chromoxide oder Eisenoxide;
- Fasern, insbesondere Glasfasern, Kohlefasern, Metallfasern, Keramikfasern, Kunststofffasern wie Polyamidfasern oder Polyethylenfasern, oder Naturfasern wie Wolle, Cellulose, Hanf oder Sisal;
- Farbstoffe;
- Trocknungsmittel, insbesondere Molekularsiebpulver, Calciumoxid, hochreaktive Isocyanate wie p-Tosylisocyanat, monomere Diisocyanate, Mono-Oxazolidine wie Incozol® 2 (von Incorez) oder Orthoameisensäureester;
- Haftvermittler, insbesondere Organoalkoxysilane, insbesondere Epoxysilane wie insbesondere 3-Glycidoxypropyltrimethoxysilan oder 3-Glycidoxypropyltriethoxysilan, (Meth)acrylosilane, Anhydridosilane, Carbamatosilane, Alkylsilane oder Iminosilane, oder oligomere Formen dieser Silane, oder Titanate;
- latente Härter oder Vernetzer, insbesondere Ketimine, Enamine, Oxazolidine oder nicht der Formel (I) entsprechende Aldimine;
- weitere Katalysatoren, welche die Reaktion der Isocyanatgruppen beschleunigen, insbesondere Salze, Seifen oder Komplexe von Zinn, Zink, Bismut, Eisen, Aluminium, Molybdän, Dioxomolybdän, Titan, Zirkonium oder Kalium, insbesondere Zinn(II)-2-ethylhexanoat, Zinn(II)-neodecanoat, Zink(II)-acetat, Zink(II)-2-ethylhexanoat, Zink(II)-laurat, Zink(II)-acetylacetonat, Aluminiumlactat, Aluminiumoleat, Diisopropoxytitan-bis-(ethylacetoacetat) oder Kaliumacetat; tertiäre Aminogruppen enthaltende Verbindungen, insbesondere N-Ethyldiisopropylamin, N,N,N',N'-Tetramethylalkylendiamine, Pentamethylalkylentriamine und höhere Homologe davon, Bis-(N,N-diethylaminoethyl)-adipat, Tris(3-dimethylaminopropyl)amin, 1,4-Diazabicyclo[2.2.2]octan (DABCO), 1,8-Diazabicyclo[5.4.0]undec-7-en (DBU), 1,5-Diazabicyclo[4.3.0]-non-5-en (DBN), N-Alkylmorpholine, N,N'-Dimethylpiperazin; stickstoffaromatische Verbindungen wie 4-Dimethylamino-pyridin, N-Methylimidazol, N-Vinylimidazol oder 1,2-Dimethylimidazol; organische Ammoniumverbindungen wie Benzyltrimethylammoniumhydroxid oder alkoxylierte tertiäre Amine; sogenannte "delayed action"-Katalysatoren, welche Modifizierungen bekannter Metall- oder Aminkatalysatoren darstellen;
- Rheologie-Modifizierer, insbesondere Verdickungsmittel, insbesondere Schichtsilikate wie Bentonite, Derivate von Rizinusöl, hydriertes Rizinusöl, Polyamide, Polyamidwachse, Polyurethane, Harnstoffverbindungen, pyrogene Kieselsäuren, Celluloseether oder hydrophob modifizierte Polyoxyethylene;
- natürliche Harze, Fette oder Öle wie Kolophonium, Schellack, Leinöl, Rizinusöl oder Sojaöl;
- nicht-reaktive Polymere, insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid oder Magnesiumhydroxid, sowie insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)-phosphat, isopropyliertes Triphenylphosphat, Mono-, Bis-oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- Additive, insbesondere Netzmittel, Verlaufmittel, Entschäumer, Entlüfter, Stabilisatoren gegen Oxidation, Wärme, Licht oder UV-Strahlung oder Biozide;
oder weitere üblicherweise in feuchtigkeitshärtenden Zusammensetzungen eingesetzte Substanzen.

Es kann sinnvoll sein, gewisse Substanzen vor dem Einmischen in die Zusammensetzung chemisch oder physikalisch zu trocknen.

Insbesondere kann die Zusammensetzung neben dem Polyamin der Formel (I) weitere latente Härter enthalten, insbesondere Polyaldimine, welche nicht der Formel (I) entsprechen, oder Oxazolidine.

In der Zusammensetzung liegt das Verhältnis zwischen Aldiminogruppen und Isocyanatgruppen bevorzugt im Bereich von 0.05 bis 1.1, besonders bevorzugt im Bereich von 0.1 bis 1.0, insbesondere im Bereich von 0.2 bis 0.9.

Die Zusammensetzung enthält bevorzugt einen Gehalt an Polyisocyanaten und Isocyanatgruppen-haltigen Polyurethanpolymeren im Bereich von 5 bis 90 Gewichts-%, insbesondere 10 bis 80 Gewichts-%.

Die Zusammensetzung wird insbesondere unter Ausschluss von Feuchtigkeit hergestellt und bei Umgebungstemperatur in feuchtigkeitsdichten Gebinden aufbewahrt. Ein geeignetes feuchtigkeitsdichtes Gebinde besteht insbesondere aus einem gegebenenfalls beschichteten Metall und/oder Kunststoff und stellt insbesondere ein Fass, ein Container, ein Hobbock, ein Eimer, ein Kanister, eine Büchse, ein Beutel, ein Schlauchbeutel, eine Kartusche oder eine Tube dar.

Die Zusammensetzung kann in Form einer einkomponentigen oder in Form einer mehrkomponentigen, insbesondere zweikomponentigen, Zusammensetzung vorliegen.

Als "einkomponentig" wird eine Zusammensetzung bezeichnet, bei welcher alle Bestandteile der Zusammensetzung im gleichen Gebinde vorliegen und welche als solche lagerstabil ist.

Als "zweikomponentig" wird eine Zusammensetzung bezeichnet, bei welcher die Bestandteile der Zusammensetzung in zwei verschiedenen Komponenten vorliegen, welche in voneinander getrennten Gebinden gelagert und erst kurz vor oder während der Applikation der Zusammensetzung miteinander vermischt werden.

Die Zusammensetzung ist bevorzugt einkomponentig. Sie ist bei geeigneter Verpackung und Aufbewahrung lagerstabil, typischerweise während mehreren Monaten bis zu einem Jahr oder länger.

Bei der Applikation der Zusammensetzung beginnt der Prozess der Aushärtung. Als Ergebnis davon entsteht die ausgehärtete Zusammensetzung.

Im Fall einer einkomponentigen Zusammensetzung wird diese als solche appliziert und beginnt darauf unter dem Einfluss von Feuchtigkeit bzw. Wasser auszuhärten. Zur Beschleunigung der Aushärtung kann der Zusammensetzung bei der Applikation eine Beschleuniger-Komponente, welche Wasser und/oder einen Katalysator enthält oder freisetzt, zugemischt werden, oder die Zusammensetzung kann nach ihrer Applikation mit einer solchen Beschleuniger-Komponente in Kontakt gebracht werden.

Im Fall einer zweikomponentigen Zusammensetzung wird diese nach dem Vermischen der beiden Komponenten appliziert und beginnt dabei durch interne Reaktion auszuhärten, wobei die Aushärtung gegebenenfalls durch die Einwirkung von externer Feuchtigkeit vervollständigt wird. Das Vermischen der beiden Komponenten kann kontinuiertlich oder batchweise mit dynamischen Mischern oder Statikmischern erfolgen.

Bei der Aushärtung reagieren die Isocyanatgruppen unter dem Einfluss von Feuchtigkeit mit den Aldiminogruppen des Polyaldimins der Formel (I) und gegebenenfalls vorhandenen weiteren blockierten Aminogruppen. Ein Teil der Isocyanatgruppen, insbesondere die gegenüber den Aldiminogruppen überschüssigen, reagieren unter dem Einfluss von Feuchtigkeit untereinander und/oder mit gegebenenfalls in der Zusammensetzung vorhandenen weiteren Reaktivgruppen, insbesondere Hydroxylgruppen oder freien Aminogruppen. Die Gesamtheit dieser zur Aushärtung der Zusammensetzung führenden Reaktionen der Isocyanatgruppen wird auch als Vernetzung bezeichnet.

Die zur Aushärtung der einkomponentigen Zusammensetzung benötigte Feuchtigkeit gelangt bevorzugt aus der Luft (Luftfeuchtigkeit) durch Diffusion in die Zusammensetzung. Dabei bildet sich an den mit Luft in Kontakt stehenden Oberflächen der Zusammensetzung eine feste Schicht ausgehärteter Zusammensetzung ("Haut"). Die Aushärtung setzt sich entlang der Diffusionsrichtung von aussen nach innen fort, wobei die Haut zunehmend dicker wird und schliesslich die ganze applizierte Zusammensetzung umfasst. Die Feuchtigkeit kann zusätzlich oder vollständig auch aus einem oder mehreren Substrat(en), auf welche(s) die Zusammensetzung appliziert wurde, in die Zusammensetzung gelangen und/oder aus einer Beschleuniger-Komponente stammen, welche der Zusammensetzung bei der Applikation zugemischt oder nach der Applikation mit dieser in Kontakt gebracht wird, beispielsweise durch Bestreichen oder Besprühen.

Die zur Vervollständigung der Aushärtung einer zweikomponentigen Zusammensetzung gegebenenfalls benötigte externe Feuchtigkeit stammt bevorzugt aus der Luft und/oder aus den Substraten.

Die Zusammensetzung wird bevorzugt bei Umgebungstemperatur appliziert, insbesondere im Bereich von etwa 0 bis 50°C, bevorzugt im Bereich von 5 bis 40°C.

Die Aushärtung der Zusammensetzung erfolgt bevorzugt ebenfalls bei Umgebungstemperatur.

Die Zusammensetzung verfügt über eine vergleichsweise lange Offenzeit.

Als "Offenzeit" wird die Zeitspanne bezeichnet, während der die Zusammensetzung verarbeitet oder nachbearbeitet werden kann, nachdem der Aushärtungsprozess begonnen hat.

Die Zeit bis zur Ausbildung einer Haut ("Hautbildungszeit") bzw. bis zur Klebefreiheit ("tack-free time") stellt dabei ein Mass für die Offenzeit dar.

Bei der Vernetzung wird ein Aldehyd der Formel (IV) freigesetzt. Dieser ist weitgehend unflüchtig und geruchlos und verbleibt zum grössten Teil in der ausgehärteten Zusammensetzung. Dort verhält er sich bzw. wirkt wie ein Weichmacher. Als solcher kann er grundsätzlich selber migrieren und/oder die Migration von Weichmachern beeinflussen. Der Aldehyd der Formel (IV) ist mit der ausgehärteten Zusammensetzung sehr gut verträglich, migriert selber kaum und löst auch keine verstärkte Migration von Weichmachern aus.

Bevorzugt stellt die Zusammensetzung einen Klebstoff oder einen Dichtstoff oder eine Beschichtung dar.

Bevorzugt ist der Klebstoff oder Dichtstoff oder die Beschichtung elastisch.

Als Klebstoff und/oder Dichtstoff ist die Zusammensetzung insbesondere geeignet für Klebe- und Abdichtungsanwendungen, insbesondere in der Bau- und Fertigungsindustrie oder im Fahrzeugbau, insbesondere für die Parkettverklebung, Anbauteilverklebung, Hohlraumversiegelung, Montage, Modulverklebung, Karosserieverklebung, Scheibenverklebung, Fugenabdichtung oder Verankerung.

Elastische Verklebungen im Fahrzeugbau sind beispielsweise das Ankleben von Teilen wie Kunststoffabdeckungen, Zierleisten, Flansche, Stosstangen, Führerkabinen oder andere Anbauteile, an die lackierte Karosserie eines Fahrzeugs, oder das Einkleben von Scheiben in die Karosserie, wobei die Fahrzeuge insbesondere Automobile, Lastkraftwagen, Busse, Schienenfahrzeuge oder Schiffe darstellen.

Als Dichtstoff ist die Zusammensetzung insbesondere geeignet für das elastische Abdichten von Fugen, Nähten oder Hohlräumen aller Art, insbesondere von Fugen am Bau wie Dilatationsfugen oder Anschlussfugen zwischen Bauteilen. Insbesondere für das Abdichten von Dilatationsfugen an Bauwerken ist ein Dichtstoff mit weichelastischen Eigenschaften besonders geeignet.

Als Beschichtung ist die Zusammensetzung geeignet zum Schutz von Böden oder Mauern, insbesondere als Beschichtung von Balkonen, Terrassen, Plätzen, Brücken, Parkdecks, oder zur Abdichtung von Dächern, insbesondere Flachdächern oder schwach geneigten Dachflächen oder Dachgärten, oder im Innern von Gebäuden zur Wasserabdichtung, beispielsweise unter Fliesen oder Keramikplatten in Nasszellen oder Küchen, oder als Bodenbelag in Küchen, Industriehallen oder Fabrikationsräumen, oder als Abdichtung in Auffangwannen, Kanälen, Schächten oder Abwasserbehandlungsanlagen, oder zum Schutz von Oberflächen als Lack oder Versiegelung, oder als Vergussmasse zur Hohlraumversiegelung, als Nahtabdichtung oder als Schutzbeschichtung für beispielsweise Rohre.

Sie kann auch zu Reparaturzwecken als Abdichtung oder Beschichtung verwendet werden, beispielsweise von undichten Dachmembranen oder nicht mehr tauglichen Bodenbelägen oder insbesondere als Reparaturmasse für hochreaktive Spritzabdichtungen.

Die Zusammensetzung kann so formuliert sein, dass sie eine pastöse Konsistenz mit strukturviskosen Eigenschaften aufweist. Eine solche Zusammensetzung wird mittels einer geeigneten Vorrichtung appliziert, beispielsweise aus handelsüblichen Kartuschen oder Fässern oder Hobbocks, beispielsweise in Form einer Raupe, wobei diese eine im Wesentlichen runde oder dreieckige Querschnittsfläche aufweisen kann.

Die Zusammensetzung kann weiterhin so formuliert sein, dass sie flüssig und sogenannt selbstverlaufend oder nur leicht thixotrop ist und zur Applikation ausgegossen werden kann. Als Beschichtung kann sie beispielsweise anschliessend flächig bis zur erwünschten Schichtdicke verteilt werden, beispielsweise mittels einer Rolle, einem Schieber, einer Zahntraufel oder einer Spachtel. Dabei wird in einem Arbeitsgang typischerweise eine Schichtdicke im Bereich von 0.5 bis 3 mm, insbesondere 1.0 bis 2.5 mm, aufgetragen.

Geeignete Substrate, welche mit der Zusammensetzung verklebt oder abgedichtet oder beschichtet werden können, sind insbesondere
- Glas, Glaskeramik, Beton, Mörtel, Faserzement, insbesondere Faserzement-Platten, Backstein, Ziegel, Gips, insbesondere Gips-Platten, oder Natursteine wie Granit oder Marmor;
- Reparatur- oder Nivelliermassen auf Basis PCC (Polymer-modifizierter Zementmörtel) oder ECC (Epoxidharz-modifizierter Zementmörtel);
- Metalle oder Legierungen wie Aluminium, Kupfer, Eisen, Stahl, Buntmetalle, inklusive oberflächenveredelte Metalle oder Legierungen wie verzinkte oder verchromte Metalle;
- Asphalt oder Bitumen;
- Leder, Textilien, Papier, Holz, mit Harzen wie Phenol-, Melamin- oder Epoxidharzen gebundene Holzwerkstoffe, Harz-Textil-Verbundwerkstoffe oder weitere sogenannte Polymer-Composites;
- Kunststoffe wie Hart- und Weich-PVC, Polycarbonat, Polystyrol, Polyester, Polyamid, PMMA, ABS, SAN, Epoxidharze, Phenolharze, PUR, POM, TPO, PE, PP, EPM oder EPDM, jeweils unbehandelt oder oberflächenbehandelt, beispielsweise mittels Plasma, Corona oder Flammen;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- Isolierschäume, insbesondere aus EPS, XPS, PUR, PIR, Steinwolle, Glaswolle oder geschäumtem Glas (Foamglas);
- beschichtete oder lackierte Substrate, insbesondere lackierte Fliesen, gestrichener Beton, pulverbeschichtete Metalle oder Legierungen oder lackierte Bleche;
- Farben oder Lacke, insbesondere Automobildecklacke.

Die Substrate können bei Bedarf vor dem Applizieren vorbehandelt werden, insbesondere durch physikalische und/oder chemische Reinigungsverfahren oder das Aufbringen eines Aktivators oder eines Primers.

Verklebt und/oder abgedichtet werden können zwei gleichartige oder zwei verschiedene Substrate.

Aus der Applikation und Aushärtung der Zusammensetzung wird ein Artikel erhalten, welcher mit der Zusammensetzung verklebt oder abgedichtet oder beschichtet ist. Dieser Artikel kann ein Bauwerk oder ein Teil davon sein, insbesondere ein Bauwerk des Hoch- oder Tiefbaus, eine Brücke, ein Dach, ein Treppenhaus oder eine Fassade, oder er kann ein industrielles Gut oder ein Konsumgut sein, insbesondere ein Fenster, ein Rohr, ein Rotorblatt einer Windkraftanlage, eine Haushaltmaschine oder ein Transportmittel wie insbesondere ein Automobil, ein Bus, ein Lastkraftwagen, ein Schienenfahrzeug, ein Schiff, ein Flugzeug oder ein Helikopter, oder ein Anbauteil davon.

Die erfindungsgemässe Zusammensetzung weist vorteilhafte Eigenschaften auf. Sie ist unter Ausschluss von Feuchtigkeit besonders lagerstabil, auch bei hochreaktiven aromatischen Isocyanatgruppen wie jenen von MDI. Sie verfügt über eine vergleichsweise lange Offenzeit, die über einen längeren Zeitraum nach der Applikation einen nahtlosen Verlauf des applizierten Materials oder eine Positionierung oder Nachjustierung der damit verbundenen Objekte ermöglicht, was beispielsweise bei grossflächigen Beschichtungen, langen Dichtstreifen oder bei der Verklebung von grossen oder komplexen Objekten wichtig ist. Die Aushärtung erfolgt schnell, blasenfrei und ohne störende Geruchsimmissionen, wobei ein ausgehärtetes Material mit guter Festigkeit, Dehnbarkeit und Elastizität entsteht, welches nicht zu Problemen mit Weichmacher-Migration wie Ausschwitzen (Bleeding), Substratverfärbung oder Spannungsrissbildung im Substrat neigt.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

### verwendete Aldehyde:

Aldehyd-1: Fraktioniertes Reaktionsgemisch erhalten aus mittels HF-BF₃ katalysierter Formylierung von C₁₀₋₁₄-Alkylbenzol, enthaltend mehrheitlich verzweigte 4-(C₁₀₋₁₄-Alkyl)benzaldehyde. (mittleres Aldehyd-Equivalentgewicht 290 g/Eq)
p-Decyloxybenzaldehyd (262.4 g/mol)
2,2-Dimethyl-3-lauroyloxypropanal (284.4 g/mol)
Benzaldehyd (106.1 g/mol)

p-tert.Butylbenzaldehyd (162.2 g/mol)
3-Phenoxybenzaldehyd (198.2 g/mol)

Aldehyd-1 ist ein Gemisch von Aldehyden der Formel (IV) und p-Decyloxybenzaldehyd ist ein Aldehyd der Formel (IV), während 2,2-Dimethyl-3-lauroyl-oxypropanal, Benzaldehyd, p-tert.Butylbenzaldehyd und 3-Phenoxybenzaldehyd nicht der Formel (IV) entsprechen.

### verwendete Amine und Abkürzugen:

- IPDA: 3-Aminomethyl-3,5,5-trimethylcyclohexylamin (Vestamin® IPD von Evonik, 170.3 g/mol)
- NBDA: 2,5(6)-Bis(aminomethyl)bicyclo[2.2.1]heptan (PRO-NBDA von Mitsui Fine Chemicals, 154.3 g/mol)
- TCD: 3(4),8(9)-Di(aminomethyl)tricyclo[5.2.1.0(2.6)]decan (TCD Diamin von Oxea, 194.3 g/mol)
- TMD: 2,2,4- und 2,4,4-Trimethylhexamethylendiamin (Vestamin® TMD von Evonik, 158.4 g/mol)
- MPMD: 1,5-Diamino-2-methylpentan (Dytek® A von Invista, 116 g/mol)
- 1,2-PDA: 1,2-Propylendiamin (von BASF, 74.1 g/mol)
- H₁₂MDA: 4,4'-Diaminodicyclohexylmethan (von BASF, 210.4 g/mol)
- T-403: Polyoxypropylen-Triamin mit einem mittleren Molekulargewicht von ca. 440 g/mol (Jeffamine® T-403, von Huntsman, Aminzahl 359 mg KOH/g)
- HDA: Hexan-1,6-diamin (von Invista, 116.2 g/mol)
- MXDA: 1,3-Bis(aminomethyl)benzol (von Mitsubishi Gas Chem., 136.2 g/mol)
- pPhDA: 1,4-Phenylendiamin (von Sigma-Aldrich, 108.2 g/mol)

### Herstellung von Polvaldiminen:

Die **Aminzahl** (inklusive Aldiminogruppen) wurde bestimmt mittels Titration (mit 0.1N HClO₄ in Essigsäure gegen Kristallviolett).

Die **Viskosität** wurde mit einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹) gemessen.

### Aldimin A1:

50.00 g Aldehyd-1 wurden in einem Rundkolben unter Stickstoffatmosphäre vorgelegt. Unter Rühren wurden 13.93 g IPDA zugegeben und anschliessend die flüchtigen Bestandteile bei 80 °C und 10 mbar Vakuum entfernt. Es wurde eine hellgelbe, geruchlose und pH-neutrale Flüssigkeit mit einer Viskosität bei 20 °C von 21.3 Pa·s und einer Aminzahl von 150.1 mg KOH/g erhalten.

### Aldimin A2:

50.00 g Aldehyd-1 wurden in einem Rundkolben unter Stickstoffatmosphäre vorgelegt. Unter Rühren wurden 12.62 g NBDA zugegeben und anschliessend die flüchtigen Bestandteile bei 80 °C und 10 mbar Vakuum entfernt. Es wurde eine hellgelbe, geruchlose und pH-neutrale Flüssigkeit mit einer Viskosität bei 20 °C von 9.8 Pa·s und einer Aminzahl von 152.3 mg KOH/g erhalten.

### Aldimin A3:

50.00 g Aldehyd-1 wurden in einem Rundkolben unter Stickstoffatmosphäre vorgelegt. Unter Rühren wurden 15.90 g TCD zugegeben und anschliessend die flüchtigen Bestandteile bei 80 °C und 10 mbar Vakuum entfernt. Es wurde eine hellgelbe, geruchlose und pH-neutrale Flüssigkeit mit einer Viskosität bei 20 °C von 19.6 Pa·s und einer Aminzahl von 144.0 mg KOH/g erhalten.

### Aldimin A4:

50.00 g Aldehyd-1 wurden in einem Rundkolben unter Stickstoffatmosphäre vorgelegt. Unter Rühren wurden 17.21 g H₁₂MDA zugegeben und anschliessend die flüchtigen Bestandteile bei 80°C und 10 mbar Vakuum entfernt. Es wurde eine hellgelbe, geruchlose und pH-neutrale Flüssigkeit mit einer Viskosität bei 20°C von 59.6 Pa·s und einer Aminzahl von 140.2 mg KOH/g erhalten.

### Aldimin A5:

50.00 g Aldehyd-1 wurden in einem Rundkolben unter Stickstoffatmosphäre vorgelegt. Unter Rühren wurden 12.95 g TMD zugegeben und anschliessend die flüchtigen Bestandteile bei 80°C und 10 mbar Vakuum entfernt. Es wurde eine hellgelbe, geruchlose und pH-neutrale Flüssigkeit mit einer Viskosität bei 20°C von 1.6 Pa·s und einer Aminzahl von 152.4 mg KOH/g erhalten.

### Aldimin A6:

50.00 g Aldehyd-1 wurden in einem Rundkolben unter Stickstoffatmosphäre vorgelegt. Unter Rühren wurden 9.51 g MPMD zugegeben und anschliessend die flüchtigen Bestandteile bei 80°C und 10 mbar Vakuum entfernt. Es wurde eine hellgelbe, geruchlose und pH-neutrale Flüssigkeit mit einer Viskosität bei 20°C von 1.2 Pa·s und einer Aminzahl von 162.0 mg KOH/g erhalten.

### Aldimin A7:

50.00 g Aldehyd-1 wurden in einem Rundkolben unter Stickstoffatmosphäre vorgelegt. Unter Rühren wurden 25.77 g T-403 zugegeben und anschliessend die flüchtigen Bestandteile bei 80°C und 10 mbar Vakuum entfernt. Es wurde eine orangegelbe, geruchlose und pH-neutrale Flüssigkeit mit einer Viskosität bei 20°C von 4.3 Pa·s und einer Aminzahl von 126.8 mg KOH/g erhalten.

### Aldimin A8 (nicht erfindungsgemäss):

5.75 g Decyloxybenzaldehyd wurden in einem Rundkolben unter Stickstoffatmosphäre vorgelegt. Unter Rühren wurden 1.78 g IPDA zugegeben und anschliessend die flüchtigen Bestandteile bei 80°C und 10 mbar Vakuum entfernt. Es wurde ein bei 20°C wachsartiger, geruchloser und pH-neutraler Feststoff mit einer Aminzahl von 163.7 mg KOH/g und einem Schmelzpunkt von 52 bis 59°C und einer Viskosität bei 80°C von 82.4 Pa·s erhalten.

### Aldimin R1:

50.00 g Aldehyd-1 wurden in einem Rundkolben unter Stickstoffatmosphäre vorgelegt. Unter Rühren wurden 13.58 g HDA-Lösung (70 Gew.-% in Wasser) zugegeben und anschliessend die flüchtigen Bestandteile bei 80°C und 10 mbar Vakuum entfernt. Es wurde eine hellgelbe, geruchlose und pH-neutrale Flüssigkeit mit einer Viskosität bei 20°C von 1.0 Pa·s und einer Aminzahl von 161.6 mg KOH/g erhalten.

### Aldimin R2:

50.00 g Aldehyd-1 wurden in einem Rundkolben unter Stickstoffatmosphäre vorgelegt. Unter Rühren wurden 11.14 g MXDA zugegeben und anschliessend die flüchtigen Bestandteile bei 80°C und 10 mbar Vakuum entfernt. Es wurde eine blassgelbe, geruchlose und pH-neutrale Flüssigkeit mit einer Viskosität bei 20°C von 2.6 Pa·s und einer Aminzahl von 155.7 mg KOH/g erhalten.

### Aldimin R3:

50.00 g Aldehyd-1 wurden in einem Rundkolben unter Stickstoffatmosphäre vorgelegt. Unter Rühren wurden 8.85 g pPhDA zugegeben und anschliessend die flüchtigen Bestandteile bei 80°C und 10 mbar Vakuum entfernt. Es wurde eine orangebraune, geruchlose und pH-neutrale Flüssigkeit mit einer Viskosität bei 20°C von 4.9 Pa·s und einer Aminzahl von 163.0 mg KOH/g erhalten.

### Aldimin R4:

48.92 2,2-Dimethyl-3-lauroyloxypropanal wurden in einem Rundkolben unter Stickstoffatmosphäre vorgelegt. Unter Rühren wurden 13.93 g IPDA zugegeben und anschliessend die flüchtigen Bestandteile bei 80°C und 10 mbar Vakuum entfernt. Es wurde eine blassgelbe, geruchlose Flüssigkeit mit einer Aminzahl von 153.0 mg KOH/g erhalten.

### Aldimin R5:

48.87 g 2,2-Dimethyl-3-lauroyloxypropanal wurden in einem Rundkolben unter Stickstoffatmosphäre vorgelegt. Unter Rühren wurden 17.21 g H₁₂MDA zugegeben und anschliessend die flüchtigen Bestandteile bei 80°C und 10 mbar Vakuum entfernt. Es wurde eine hellgelbe, geruchlose Flüssigkeit mit einer Viskosität bei 20°C von 0.6 Pa·s und einer Aminzahl von 145.4 mg KOH/g erhalten.

### Aldimin R6:

33.43 g Benzaldehyd wurden in einem Rundkolben unter Stickstoffatmosphäre vorgelegt. Unter Rühren wurden 25.55 g IPDA zugegeben und anschliessend die flüchtigen Bestandteile bei 80°C und 10 mbar Vakuum entfernt. Erhalten wurde eine hellgelbe, geruchsintensive Flüssigkeit mit einer Aminzahl von 314.1 mg KOH/g, welche nach einigen Tagen kristallisierte.

### Aldimin R7:

27.87 g p-tert.Butylbenzaldehyd wurden in einem Rundkolben unter Stickstoffatmosphäre vorgelegt. Unter Rühren wurden 13.93 g IPDA zugegeben und anschliessend die flüchtigen Bestandteile bei 80°C und 10 mbar Vakuum entfernt. Erhalten wurde eine hellgelbe, geruchsintensive, hochviskose Flüssigkeit mit einer Aminzahl von 236.2 mg KOH/g, welche nach einigen Tagen kristallisierte. Die Viskosität bei 80°C betrug 23.7 Pa·s.

### Aldimin R8:

34.06 g 3-Phenoxybenzaldehyd wurden in einem Rundkolben unter Stickstoffatmosphäre vorgelegt. Unter Rühren wurden 13.93 g IPDA zugegeben und anschliessend die flüchtigen Bestandteile bei 80°C und 10 mbar Vakuum entfernt. Erhalten wurde eine hellgelbe, geruchsarme, hochviskose Flüssigkeit mit einer Aminzahl von 203.8 mg KOH/g, welche nach einigen Tagen kristallisierte. Die Viskosität bei 80°C betrug 5.1 Pa·s.

Die Aldimine **A1** bis **A8** sind Polyaldimine der Formel (I). Die Aldimine **R1** bis **R8** sind Vergleichsbeispiele.

### Herstellung von Isocyanatgruppen-haltigen Polymeren

### Polymer P1:

4000 g Polyoxypropylen-Diol (Acclaim® 4200, von Covestro; OH-Zahl 28.5 mg KOH/g) und 520 g 4,4'-Diphenylmethandiisocyanat (Desmodur® 44 MC L, von Covestro) wurden nach bekanntem Verfahren bei 80°C zu einem bei Raumtemperatur flüssigen, NCO-terminierten Polyurethanpolymer mit einem Gehalt an freien Isocyanatgruppen von 1.85 Gewichts-% umgesetzt.

### Polymer P2:

3080 g Polyoxypropylen-Diol (Acclaim® 4200, von Covestro; OH-Zahl 28.5 mg KOH/g), 1540 g Polyoxypropylenpolyoxyethylen-Triol (Caradol® MD34-02, von Shell; OH-Zahl 35.0 mg KOH/g) und 385 g Toluylendiisocyanat (Desmodur® T 80 P, Covestro) wurden bei 80°C nach bekanntem Verfahren zu einem bei Raumtemperatur flüssigen, NCO-terminierten Polyurethanpolymer mit einem Gehalt an freien Isocyanatgruppen von 1.50 Gew.-% umgesetzt.

### Polymer P3:

590 g Polyoxypropylen-Diol (Acclaim® 4200, von Covestro; OH-Zahl 28.5 mg KOH/g), 1180 g Polyoxypropylenpolyoxyethylen-Triol (Caradol® MD34-02, von Shell; OH-Zahl 35.0 mg KOH/g) und 230 g Isophorondiisocyanat (Vestanat® IPDI, Degussa) wurden nach bekanntem Verfahren bei 80 °C zu einem bei Raumtemperatur flüssigen, NCO-terminierten Polyurethanpolymer mit einem Gehalt an freien Isocyanatgruppen von 2.10 Gewichts-% umgesetzt.

### Polymer P4:

300.0 g Polyoxypropylenpolyoxyethylen-Diol (Desmophen® L300, von Covestro; OH-Zahl 190.0 mg KOH/g) und 228.8 g Isophorondiisocyanat (Vestanat® IPDI, Degussa) wurden nach bekanntem Verfahren bei 60°C zu einem bei Raumtemperatur flüssigen, NCO-terminierten Polyurethanpolymer mit einem Gehalt an freien Isocyanatgruppen von 8.35 Gewichts-% umgesetzt.

### Einkomponentige Zusammensetzungen

### Zusammensetzungen Z1 bis Z15 und Ref1 bis Ref10

Für jede Zusammensetzung wurden die in den Tabellen 1 bis 3 angegebenen Inhaltsstoffe in den angegebenen Mengen (in Gewichtsteilen) mittels eines Zentrifugalmischers (SpeedMixer™ DAC 150, FlackTek Inc.) unter Feuchtigkeitsausschluss während einer Minute bei 3000 rpm vermischt und unter Feuchtigkeitsausschluss aufbewahrt. Jede Zusammensetzung wurde folgendermassen geprüft:
Als Mass für die Lagerstabilität wurden die **Viskosität (1d RT)** am Folgetag der Herstellung und die **Viskosität (7d 60°C)** nach einer Lagerung im verschlossenen Gebinde während 7 Tagen in einem 60°C Umluftofen bestimmt. Die Viskosität wurde jeweils bei einer Temperatur von 20°C mit einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹) gemessen. Ein starker Anstieg der Viskosität bei der Lagerung zeigt eine ungenügende Lagerstabilität.

Als Mass für die Offenzeit wurde die Hautbildungszeit **(tack-free time)** bestimmt. Dazu wurden einige Gramm der Zusammensetzung in einer Schichtdicke von ca. 2 mm auf Pappkarton aufgetragen und im Normklima die Zeitdauer bestimmt, bis beim leichten Antippen der Oberfläche der Zusammensetzung mittels einer Pipette aus LDPE erstmals keine Rückstände auf der Pipette mehr zurückblieben.

Zur Bestimmung der mechanischen Eigenschaften wurde jede Zusammensetzung auf eine PTFE-beschichtete Folie zu einem Film von 2 mm Dicke ausgegossen, während 7 Tagen im Normklima gelagert, einige Hanteln mit einer Länge von 75 mm bei einer Steglänge von 30 mm und einer Stegbreite von 4 mm aus dem Film ausgestanzt und diese gemäss DIN EN 53504 bei einer Zuggeschwindigkeit von 200 mm/min auf **Zugfestigkeit** (Bruchkraft), **Bruchdehnung, E-Modul** 5% (bei 0.5-5% Dehnung) und **E-Modul 50%** (bei 0.5 - 50% Dehnung) geprüft.

Der **Aspekt** wurde visuell an den hergestellten Filmen beurteilt. Als "schön" wurde ein klarer Film mit nichtklebriger Oberfläche ohne Blasen bezeichnet. Der **Geruch** wurde durch Riechen mit der Nase im Abstand von 2 cm an den frisch hergestellten Filmen beurteilt. "Nein" bedeutet, dass kein Geruch wahrnehmbar war.

Die Resultate sind in den Tabellen 1 bis 3 angegeben.

Bei den Zusammensetzungen **Z1** bis **Z15** handelt es sich um erfindungsgemässe Beispiele. Bei den Zusammensetzungen **Ref1** bis **Ref10** handelt es sich um Vergleichsbeispiele.

**Tabelle 1: Zusammensetzung (in Gewichtsteilen) und Eigenschaften von Z1 bis Z7 und Ref1 bis Ref8.**

| **Zusammensetzung** | **Z1** | **Z2** | **Z3** | **Z4** | **Z5** | **Z6** | **Z7 Ref.** |
|---|---|---|---|---|---|---|---|
| **Polymer P1** | 80.00 | 80.00 | 80.00 | 80.00 | 80.00 | 80.00 | 80.00 |
| **Aldimin** | **A1** | **A2** | **A3** | **A4** | **A5** | **A6** | **A8** |
| | 9.20 | 8.98 | 9.48 | 9.67 | 9.03 | 8.52 | 8.44² |
| Salicylsäurelösung¹ | 1.50 | 1.50 | 1.50 | 1.50 | 1.50 | 1.50 | 1.50 |
| Viskosität (1d RT) | 29.4 | 43.1 | 47.8 | 32.0 | 43.4 | 41.7 | 34.8 |
| [Pa·s] (7d 60°C) | 34.8 | 65.3 | 69.5 | 37.5 | 50.3 | 57.9 | 42.3 |
| tack-free time | 1h 20' | 1h | 1h 13' | 1h 10' | 1h 40' | 1h 5' | 2h 10' |
| Zugfestigkeit [MPa] | 1.62 | 1.76 | 2.01 | 3.46 | 1.60 | 1.90 | 2.62 |
| Bruchdehnung [%] | 1240 | 731 | 876 | 803 | 1373 | 942 | 1289 |
| E-Modul 5% [MPa] | 0.75 | 0.94 | 0.97 | 1.27 | 0.61 | 0.85 | 1.03 |
| E-Modul 50% | 0.41 | 0.55 | 0.58 | 0.75 | 0.35 | 0.45 | 0.57 |
| Aspekt | schön | schön | schön | schön | schön | schön | schön |
| Geruch | nein | nein | nein | nein | nein | nein | nein |

| | | | | | | | |
|---|---|---|---|---|---|---|---|
| ¹ 5% in Dioctyladipat ² bei 80°C aufgeschmolzen | | | | | | | |

**Tabelle 1: (Fortsetzung)**

| **Zusammensetzung** | **Ref1** | **Ref2** | **Ref3** | **Ref4** | **Ref5** | **Ref6** | **Ref7** | **Ref8** |
|---|---|---|---|---|---|---|---|---|
| **Polymer P1** | 80.00 | 80.00 | 80.00 | 80.00 | 80.00 | 80.00 | 80.00 | 80.00 |
| **Aldimin** | **R1** | **R2** | **R3** | **R4** | **R5** | **R6** | **R7** | **R8** |
| | 8.56 | 8.76 | 8.42 | 9.03 | 9.51 | 4.40 | 5.85 | 6.78 |
| Salicylsäurelösung¹ | 1.50 | 1.50 | 1.50 | 1.50 | 0.20 | 1.50 | 1.50 | 1.50 |
| Viskosität (1d RT) | 50.0 | 50.5 | 35.7 | 22.1 | 28.6 | 36.5 | 43.8 | 39.9 |
| [Pa·s] (7d 60°C) | 87.2 | 137.5 | 41.1 | 27.0 | 33.5 | 41.1 | 47.4 | 44.6 |
| tack-free time | 1h 10' | 35' | 1h 30' | 45' | 1h 22' | 1h 30' | 2h | 1h 35' |
| Zugfestigkeit [MPa] | 2.91 | 3.47 | 2.10 | 1.18 | 1.62 | 1.66 | 1.44 | 0.55 |
| Bruchdehnung [%] | 508 | 823 | 248 | 1240 | 299 | 936 | 1674 | 1098 |
| E-Modul 5% [MPa] | 5.66 | 6.72 | 10.30 | 1.02 | 1.61 | 1.00 | 0.79 | 0.41 |
| E-Modul 50% | 1.82 | 1.70 | 1.81 | 0.54 | 0.95 | 0.51 | 0.40 | 0.17 |
| Aspekt | schön | schön | schön | schön | schön | schön | schön | schön |
| Geruch | nein | nein | nein | nein | nein | stark | stark | leicht |

**Tabelle 2: Zusammensetzung (in Gewichtsteilen) und Eigenschaften von Z8 bis Z11 und Ref9 bis Ref13. ¹ 5% in Dioctyladipat**

| **Zusammensetzung** | **Z8** | **Z9** | **Z10** | **Z11** | **Ref9** | **Ref10** | **Ref11** | **Ref12** | **Ref13** |
|---|---|---|---|---|---|---|---|---|---|
| **Polymer P2** | 80.00 | 80.00 | 80.00 | 80.00 | 80.00 | 80.00 | 80.00 | 80.00 | 80.00 |
| **Aldimin** | **A1** | **A4** | **A6** | **A7** | **R1** | **R2** | **R3** | **R4** | **R5** |
| | 7.53 | 7.91 | 6.97 | 8.92 | 7.00 | 7.17 | 6.89 | 7.39 | 7.78 |
| Salicylsäurelösung¹ | 1.50 | 1.50 | 1.50 | 1.50 | 1.50 | 1.50 | 1.50 | 0.20 | 0.20 |
| Viskosität (1d RT) | 16.3 | 24.1 | 24.9 | 16.5 | 19.4 | 27.6 | 23.7 | 27.5 | 27.9 |
| [Pa·s] (7d 60°C) | 20.6 | 33.0 | 38.1 | 23.1 | 28.0 | 45.3 | 30.0 | 48.5 | 50.1 |
| tack-free time | 1h 35' | 55' | 50' | 50' | 45' | 40' | 2h 35' | 1h 20' | 63' |
| Zugfestigkeit [MPa] | 0.52 | 1.05 | 0.66 | 0.65 | 1.22 | 1.72 | 1.79 | 0.66 | 0.92 |
| Bruchdehnung [%] | 297 | 288 | 241 | 115 | 373 | 346 | 241 | 214 | 211 |
| E-Modul 5% [MPa] | 0.41 | 0.98 | 0.66 | 0.82 | 1.68 | 3.54 | 4.60 | 0.69 | 1.06 |
| E-Modul 50% | 0.21 | 0.57 | 0.40 | 0.62 | 0.74 | 1.07 | 1.42 | 0.44 | 0.66 |
| Aspekt | schön | schön | schön | schön | schön | schön | schön | schön | schön |
| Geruch | nein | nein | nein | nein | nein | nein | nein | nein | nein |

**Tabelle 3: Zusammensetzung (in Gewichtsteilen) und Eigenschaften von Z12 bis Z15. ¹ 5% in Dioctyladipat**

| **Zusammensetzung** | **Z12** | **Z13** | **Z14** | **Z15** |
|---|---|---|---|---|
| **Polymer P3** | 80.00 | 80.00 | 80.00 | 80.00 |
| **Aldimin** | **A1** | **A4** | **A6** | **A7** |
| | 10.47 | 10.99 | 9.68 | 12.39 |
| Salicylsäurelösung¹ | 1.50 | 1.50 | 1.50 | 1.50 |
| Viskosität (1d RT) | 15.2 | 14.7 | 13.4 | 15.1 |
| [Pa·s] (7d 60°C) | 17.5 | 17.5 | 17.3 | 17.5 |
| tack-free time | 3h | 2h 15' | 2h 5' | 1h 20' |
| Zugfestigkeit [MPa] | 1.42 | 1.69 | 0.94 | 0.94 |
| Bruchdehnung [%] | 240 | 263 | 264 | 128 |
| E-Modul 5% [MPa] | 1.72 | 1.56 | 0.85 | 0.90 |
| E-Modul 50% | 0.82 | 0.96 | 0.52 | 0.87 |
| Aspekt | schön | schön | schön | schön |
| Geruch | nein | nein | nein | nein |

### Zusammensetzungen Z16 und Ref14

Für jede Zusammensetzung wurden die in der Tabelle 4 angegebenen Inhaltsstoffe in den angegebenen Mengen (in Gewichtsteilen) mittels eines Zentrifugalmischers (SpeedMixer™ DAC 150, FlackTek Inc.) unter Feuchtigkeitsausschluss während einer Minute bei 3000 rpm vermischt und unter Feuchtigkeitsausschluss aufbewahrt. Jede Zusammensetzung wurde folgendermassen geprüft:
Als Mass für die Weichmacher-Migration wurde jede Zusammensetzung so auf eine Kartonunterlage appliziert, dass sie eine runde Grundfläche von 12 mm Durchmesser und eine Höhe von 20 mm aufwies, und während 7 Tagen im NK gelagert. Um jede Zusammensetzung herum war danach auf dem Karton ein dunkler ovaler Fleck entstanden. Dessen Ausmasse (Höhe und Breite) wurden ausgemessen und in der Tabelle 4 als **Migration** angegeben.

Bei der Zusammensetzung **Z16** handelt es sich um ein erfindungsgemässes Beispiel. Bei der Zusammensetzung **Ref14** handelt es sich um ein Vergleichsbeispiel.

**Tabelle 4: Zusammensetzung (in Gewichtsteilen) und Eigenschaften von Z16 und Ref14.**

| **Zusammensetzung** | | **Z16** | **Ref14** |
|---|---|---|---|
| **Polymer P4** | | 15.00 | 15.00 |
| Kreide¹ | | 15.00 | 15.00 |
| Kieselsäure² | | 1.13 | 1.13 |
| **Aldimin** | | **A1** | **R4** |
| | | 7.80 | 7.65 |
| Dibutylzinndilaurat-Lösung³ | | 1.50 | 1.50 |
| Salicylsäure-Lösung⁴ | | 3.00 | 3.00 |
| Migration | Höhe | 80 | 110 |
| [mm] | Breite | 55 | 85 |

| | | | |
|---|---|---|---|
| ¹ gemahlenes, mit Fettsäure beschichtetes Calciumcarbonat ² hydrophob modifizierte pyrogene Kieselsäure ³ 5% in Diisodecylphthalat ⁴ 5% in Dioctyladipat | | | |

### Zusammensetzungen Z17 und Ref15 bis Ref16

Diese Zusammensetzungen wurden anhand der Angaben in der Tabelle 5 auf die gleiche Weise wie für Zusammensetzung **Z16** beschrieben hergestellt.

Als Mass für die Weichmacher-Migration wurde jede Zusammensetzung so auf eine Kartonunterlage appliziert, dass sie eine runde Grundfläche von 15 mm Durchmesser und eine Höhe von 4 mm aufwies, und während 3 Monaten im NK gelagert. Um jede Zusammensetzung herum entstand auf dem Karton ein dunkler ovaler Fleck, dessen Ausmasse (Höhe und Breite) nach 7 Tagen und nach 3 Monaten im NK ausgemessen und in der Tabelle 5 als **Migration** (7d) bzw. (3 Monate) angegeben wurden.

Als Mass für die Neigung zu **Spannungsrissbildung** auf Kunststoff wurde jede Zusammensetzung auf eine durchsichtige, vorgespannte Kunststoffplatte aus Polycarbonat (Makrolon®) mit den Massen 150 x 30 x 2 mm so aufgetragen, dass in der Mitte der gespannten Platte eine Beschichtung von 30 x 30 x 2 mm entstand. Nach 24 h im Normklima wurde die Beschichtung bzw. die Zusammensetzung entfernt und die Platte auf Rissbildung und sonstige optische Veränderungen untersucht. Die vorgespannte Kunststoffplatte war dabei jeweils so über einem auf einem Brett angebrachten Rundholz von 12.5 mm Duchmesser fixiert, dass die lange Seite quer zum Rundholz lag und die schmalen Enden auf dem Brett fixiert waren. Als "wenig" wird die Bildung von kleinen, schwach sichtbaren Risschen von 2 bis 3 mm Länge im Randbereich der Platte bezeichnet, welche nur oberflächlich vorhanden waren. Als "stark" wird ein kompletter Riss über die ganze Breite der Platte bezeichnet, der über die gesamte Dicke der Platte sichtbar war. Zusätzlich waren dabei sehr viele kleine Risse im Randbereich der Platte vorhanden.

Bei der Zusammensetzung **Z17** handelt es sich um ein erfindungsgemässes Beispiel. Bei den Zusammensetzungen **Ref15** bis **Ref16** handelt es sich um Vergleichsbeispiele.

**Tabelle 5: Zusammensetzung (in Gewichtsteilen) und Eigenschaften von Z17 und Ref15 bis Ref16.**

| **Zusammensetzung** | | **Z17** | **Ref15** | **Ref16** |
|---|---|---|---|---|
| **Polymer P4** | | 15.00 | 15.00 | 15.00 |
| Kreide | | 15.00 | 15.00 | 15.00 |
| Kieselsäure | | 1.13 | 1.13 | 1.13 |
| **Aldimin** | | **A1** | **R4** | - |
| | | 5.57 | 5.46 | |
| Dibutylzinndilaurat-Lösung¹ | | 1.50 | 1.50 | 1.50 |
| Salicylsäure-Lösung² | | 3.00 | 3.00 | 3.00 |
| **Migration** | Höhe | 18 | 37 | 17 |
| **(7d)** [mm] | Breite | 19 | 41 | 17 |
| **Migration** | Höhe | 23 | 47 | 25 |
| **(3 Monate)** [mm] | Breite | 23 | 52 | 25 |
| **Spannungsrissbildung** | | wenig | stark | wenig |

| | | | | |
|---|---|---|---|---|
| ¹ 5% in Diisodecylphthalat ² 5% in Dioctyladipat | | | | |

Aus den Resultaten zur Migration der Tabelle 5 ist ersichtlich, dass die erfindungsgemässe Zusammensetzung **Z17** mit Aldimin **A1** eine ähnliche bis sogar geringere Neigung zu Weichmachermigration aufweist im Vergleich zur Zusammensetzung **Ref16** ohne Aldimin. Im Gegensatz dazu zeigt die Zusammensetzung **Ref15** mit Aldimin **R4,** welches ebenfalls einen langkettigen Substituenten enthält und ein ähnlich hohes Molekulargewicht wie Aldimin **A1** aufweist, eine starke Weichmachermigration.

## Patentansprüche

1. Polyaldimin der Formel (I), 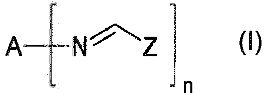 wobei
n für 2 oder 3 steht,
Z für einen mit einer Alkyl- und/oder Alkoxy-Gruppe substituierten Aryl-Rest mit insgesamt 12 bis 26 C-Atomen steht, und
A für einen n-wertigen aliphatischen oder cycloaliphatischen, gegebenenfalls Ether-Sauerstoff aufweisenden Kohlenwasserstoff-Rest mit einem Molekulargewicht im Bereich von 42 bis 6'000 g/mol, welcher über mindestens ein tertiäres oder quartäres C-Atom gebunden ist und/oder ein bi- oder tricyclisches Ringsystem enthält, steht,
**dadurch gekennzeichnet, dass** Z für einen Rest der Formel (II) steht, 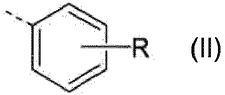
wobei R für einen verzweigten Alkyl-Rest mit 10 bis 14 C-Atomen steht.

2. Polyaldimin gemäss Anspruch1, **dadurch gekennzeichnet, dass** R für einen Rest der Formel 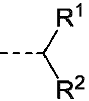 steht, wobei R¹ und R² jeweils für einen Alkyl-Rest stehen und zusammen 9 bis 13 C-Atome aufweisen.

3. Polyaldimin gemäss einem der Ansprüche 1 oder 2, **dadurch gekennzeichnet, dass** A ausgewählt ist aus der Gruppe bestehend aus 1,2-Propylen, 1,3-Pentylen, 2-Methyl-1,5-pentylen, 2,2(4),4-Trimethyl-1,6-hexamethylen, 1,2-Cyclohexylen, (1,5,5-Trimethylcyclohexan-1-yl)methan-1,3, 4(2)-Methyl-1,3-cyclohexylen, Methylendicyclohexan-4-yl, Methylenbis(2-methylcyclohexan-4-yl), (Bicyclo[2.2.1]heptan-2,5(2,6)-diyl)dimethylen, (Tricyclo[5.2.1.0^{2,6}]decan-3(4),8(9)-diyl)dimethylen, α,ω-Polyoxypropylen mit einem mittleren Molekulargewicht im Bereich von 170 bis 5'000 g/mol und Trimethylolpropan- oder Glycerin-gestartetem Tris(ω-polyoxypropylen) mit einem mittleren Molekulargewicht im Bereich von 330 bis 6'000 g/mol.

4. Gemisch aus Polyaldiminen gemäss einem der Ansprüche 1 bis 3, bei welchen Z jeweils für einen Rest der Formel (II) steht und R dabei ausgewählt ist aus verzweigten Decyl-, Undecyl-, Dodecyl-, Tridecyl- und Tetradecyl-Resten.

5. Verwendung von mindestens einem Polyaldimin gemäss einem der Ansprüche 1 bis 4 als latenter Härter für Isocyanatgruppen-haltige Zusammensetzungen.

6. Verwendung gemäss Anspruch 5, **dadurch gekennzeichnet, dass** die Isocyanatgruppen teilweise oder vollständig abgeleitet sind von 4,4'- oder 2,4'- oder 2,2'-Diphenylmethandiisocyanat oder beliebigen Gemischen dieser Isomeren.

7. Zusammensetzung umfassend
- mindestens ein Polyaldimin gemäss einem der Ansprüche 1 bis 4 und
- mindestens ein Polyisocyanat und/oder mindestens ein Isocyanatgruppen-haltiges Polyurethanpolymer.

8. Zusammensetzung gemäss Anspruch 7, **dadurch gekennzeichnet, dass** sie mindestens ein Isocyanatgruppen-haltiges Polyurethanpolymer enthält.

9. Zusammensetzung gemäss Anspruch 8, **dadurch gekennzeichnet, dass** die Isocyanatgruppen teilweise oder vollständig abgeleitet sind von 4,4'- oder 2,4'- oder 2,2'-Diphenylmethandiisocyanat oder beliebigen Gemischen dieser Isomeren.

10. Zusammensetzung gemäss einem der Ansprüche 7 bis 9, **dadurch gekennzeichnet, dass** sie zusätzlich mindestens einen weiteren Bestandteil ausgewählt aus Katalysatoren, Füllstoffen, Weichmachern und Lösemitteln enthält.

11. Zusammensetzung gemäss einem der Ansprüche 7 bis 10, **dadurch gekennzeichnet, dass** sie einkomponentig ist.

12. Zusammensetzung gemäss einem der Ansprüche 7 bis 11, **dadurch gekennzeichnet, dass** sie einen Klebstoff oder einen Dichtstoff oder eine Beschichtung darstellt.

## Claims

1. Polyaldimine of the formula (I) 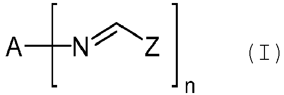 where
n is 2 or 3,
Z is an aryl radical substituted by an alkyl and/or alkoxy group and having a total of 12 to 26 carbon atoms, and A is an n-valent aliphatic or cycloaliphatic hydrocarbyl radical optionally containing ether oxygen and having a molecular weight in the range from 42 to 6'000 g/mol, bonded via at least one tertiary or quaternary carbon atom and/or containing a bi- or tricyclic ring system, **characterized in that** Z is a radical of the formula (II) 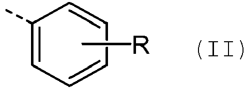
where R is a branched alkyl radical having 10 to 14 carbon atoms.

2. Polyaldimine according to Claim 1, **characterized in that** R is a radical of the formula 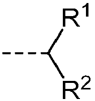 where R¹ and R² are each an alkyl radical and together have 9 to 13 carbon atoms.

3. Polyaldimine according to either of Claims 1 and 2, **characterized in that** A is selected from the group consisting of 1,2-propylene, 1,3-pentylene, 2-methyl-1,5-pentylene, 2,2(4),4-trimethyl-1,6-hexamethylene, 1,2-cyclohexylene, (1,5,5-trimethylcyclohexan-1-yl)methane-1,3, 4(2)-methyl-1,3-cyclohexylene, methylenedicyclohexan-4-yl, methylenebis(2-methylcyclohexan-4-yl), (bicyclo[2.2.1]heptan-2,5(2,6)-diyl)dimethylene, (tricyclo[5.2.1.0^{2,6}]decane-3(4),8(9)-diyl)dimethylene, α,ω-polyoxypropylene having an average molecular weight in the range from 170 to 5'000 g/mol and trimethylolpropane- or glycerol-started tris(ω-polyoxypropylene) having an average molecular weight in the range from 330 to 6'000 g/mol.

4. Mixture of polyaldimines according to any of Claims 1 to 3, in which each Z is a radical of the formula (II) and R is selected from branched decyl, undecyl, dodecyl, tridecyl and tetradecyl radicals.

5. Use of at least one polyaldimine according to any of Claims 1 to 4 as latent hardener for compositions containing isocyanate groups.

6. Use according to Claim 5, **characterized in that** some or all of the isocyanate groups are derived from 4,4'- or 2,4'- or 2,2'-diphenylmethane diisocyanate or any mixtures of these isomers.

7. Composition comprising
- at least one polyaldimine according to any of Claims 1 to 4 and
- at least one polyisocyanate and/or at least one polyurethane polymer containing isocyanate groups.

8. Composition according to Claim 7, **characterized in that** it comprises at least one polyurethane polymer containing isocyanate groups.

9. Composition according to Claim 8, **characterized in that** some or all of the isocyanate groups are derived from 4,4'- or 2,4'- or 2,2'-diphenylmethane diisocyanate or any mixtures of these isomers.

10. Composition according to any of Claims 7 to 9, **characterized in that** it additionally comprises at least one further constituent selected from catalysts, fillers, plasticizers and solvents.

11. Composition according to any of Claims 7 to 10, **characterized in that** it is a one-component composition.

12. Composition according to any of Claims 7 to 11, **characterized in that** it is an adhesive or a sealant or a coating.

## Revendications

1. Polyaldimine de formule (I) 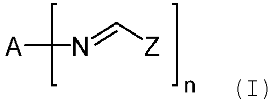 dans laquelle
n représente 2 ou 3,
Z représente un radical aryle substitué par un groupe alkyle et/ou alcoxy et ayant un total de 12 à 26 atomes de carbone, et
A représente un radical hydrocarbyle aliphatique ou cycloaliphatique n-valent contenant éventuellement un oxygène d'éther et ayant une masse moléculaire dans la plage de 42 à 6 000 g/mol, lié par l'intermédiaire d'au moins un atome de carbone tertiaire ou quaternaire et/ou contenant un système cyclique bi- ou tricyclique, **caractérisée en ce que** Z représente un radical de formule (II) 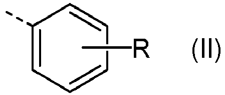
dans laquelle R représente un radical alkyle ramifié ayant 10 à 14 atomes de carbone.

2. Polyaldimine selon la revendication 1, **caractérisée en ce que** R est un radical de formule 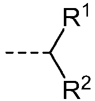 dans laquelle R¹ et R² représentent chacun un radical alkyle et ont ensemble 9 à 13 atomes de carbone.

3. Polyaldimine selon l'une des revendications 1 ou 2, **caractérisée en ce que** A est choisi dans le groupe consistant en le 1,2-propylène, le 1,3-pentylène, le 2-méthyl-1,5-pentylène, le 2,2(4),4-triméthyl-1,6-hexaméthylène, le 1,2-cyclohexylène, le (1,5,5-triméthylcyclohexan-1-yl)méthane-1,3, le 4(2)-méthyl-1,3-cyclohexylène, un méthylènedicyclohexan-4-yle, un méthylènebis(2-méthylcyclohexan-4-yle), le (bicyclo[2.2.1]heptan-2,5 (2,6)-diyl)diméthylène, le (tricyclo[5.2.1.0^{2,6}] décane-3(4),8(9)-diyl)diméthylène, un α,ω-polyoxypropylène ayant une masse moléculaire moyenne dans la plage de 170 à 5 000 g/mol et un tris(ω-polyoxypropylène) obtenu à partir de triméthylolpropane ou de glycérol ayant une masse moléculaire moyenne dans la plage de 330 à 6 000 g/mol.

4. Mélange de polyaldimines selon l'une des revendications 1 à 3, dans lequel chaque Z représente un radical de formule (II) et R est choisi parmi les radicaux décyle, undécyle, dodécyle, tridécyle et tétradécyle ramifiés.

5. Utilisation d'au moins une polyaldimine selon l'une des revendications 1 à 4 en tant que durcisseur latent pour des compositions contenant des groupes isocyanate.

6. Utilisation selon la revendication 5, **caractérisée en ce que** certains ou tous les groupes isocyanate dérivent du diisocyanate de 4,4'- ou de 2,4'- ou de 2,2'-diphénylméthane ou de tous mélanges de ces isomères.

7. Composition comprenant
- au moins une polyaldimine selon l'une des revendications 1 à 4 et
- au moins un polyisocyanate et/ou au moins un polymère de polyuréthane contenant des groupes isocyanate.

8. Composition selon la revendication 7, **caractérisée en ce qu'**elle comprend au moins un polymère de polyuréthane contenant des groupes isocyanate.

9. Composition selon la revendication 8, **caractérisée en ce que** certains ou tous les groupes isocyanate dérivent du diisocyanate de 4,4'- ou de 2,4'- ou de 2,2'-diphénylméthane ou de tous mélanges de ces isomères.

10. Composition selon l'une des revendications 7 à 9, **caractérisée en ce qu'**elle comprend en outre au moins un constituant supplémentaire choisi parmi les catalyseurs, les charges, les plastifiants et les solvants.

11. Composition selon l'une des revendications 7 à 10, **caractérisée en ce qu'**il s'agit d'une composition mono-composant.

12. Composition selon l'une des revendications 7 à 11, **caractérisée en ce qu'**elle est un adhésif ou un produit d'étanchéité ou un revêtement.
